# EUROPEAN PATENT APPLICATION

(11) **EP 4 151 711 A1**
(43) Date of publication of application: **22.03.2023**
(21) Application number: 21802928.8
(22) Date of filing: 19.02.2021
(51) Int. Cl.: C12M 1/00, C12Q 1/02

(54) **CELL DETECTION DEVICE AND CELL DETECTION METHOD**

(30) Priority: 14.05.2020 JP 2020085387
(71) Applicant: HITACHI HIGH-TECH CORPORATION, Tokyo 105-6409 (JP)
(72) Inventor: ISHIMARU Masako, Tokyo 100-8280 (JP); NODA Hideyuki, Tokyo 100-8280 (JP)
(74) Representative: Mewburn Ellis LLP
(86) International application number: PCT/JP2021/006273
(87) International publication number: WO 2021/229884

(57) **Abstract**

Provided is a technique for detecting a cell-derived substance with high sensitivity without destroying cells. A cell detection device of the present disclosure includes: a medium adding device configured to add a part of a prepared culture medium to a culture container holding cells to be detected; a medium collection device configured to collect the culture medium from the culture container; and a detection device configured to detect light from the luminescent reagent mixed with the collected culture medium.

## Description

### Technical Field

The present disclosure relates to a cell detection device and a cell detection method.

### Background Art

In a sterility test for pharmaceuticals and the like, it is necessary to detect a very small number of bacteria or fungi (hereinafter, simply referred to as "bacteria"). A conventional culturing method used for a sterility test is a method of culturing bacteria in a culture medium to increase the number of cells and detecting the bacteria. Because of this, there has been a major problem that culture for one day or more is required and it takes time to obtain a sufficient number of bacteria for detection. Therefore, in recent years, several rapid test methods have been developed.

Among them, a detection method by an ATP (Adenosine Triphosphate) method is known as a method capable of detecting bacteria with high sensitivity. The ATP method is a method for detecting ATP of bacteria by bioluminescence by a luciferin-luciferase reaction, and it is generally possible to detect bacteria with about 100 CFU (Colony forming unit).

For example, PTL 1 discloses detecting bacterial proliferation and killing with high sensitivity by dispensing a bacterial culture solution and a luminescent reagent into a plate and by performing luminescence measurement based on an ATP method.

In addition, PTL 2 discloses a method of filtering a sample through a filter to collect bacteria on the filter and concentrating the bacteria, further removing extracellular ATP to remove background ATP, and then extracting intracellular ATP. In the method of PTL 2, bacteria can be detected with high sensitivity, and bacteria can be detected from several bacteria.

### Citation List

### Patent Literature

PTL 1: JP 2696081 B2
PTL 2: JP 2013-116083 A

### Summary of Invention

### Technical Problem

On the other hand, when bacteria are detected in the test of presence or absence of bacteria, it is desirable to perform other tests such as identification of bacterial species using the same sample. This is because details of bacteria mixed in the sample are grasped to estimate a contamination path of bacteria and to reflect the contamination path in quality control. However, in the method for measuring intracellular ATP as described above, it is necessary to destroy bacteria when extracting the intracellular ATP. Therefore, it is not possible to perform enrichment culture thereafter, and it is not possible to perform an identification test or the like.

In order to further enrich and culture bacteria after detecting of the presence of bacteria, it is conceivable to adopt a method in which bacteria are partially broken and examined with an ATP method. That is, this is a method in which bacteria are proliferated in a liquid culture medium, a part of the liquid culture medium is fractionated every time, and intracellular ATP is measured, and the bacteria are regarded as being proliferated when an increase in ATP is observed. In this method, viable bacteria remain in the original liquid culture medium. Thus, it is possible to proceed to another test by performing enrichment culture thereafter. However, since fractionating is repeated from the liquid culture medium, it is necessary to start culture with a culture medium volume in consideration of the maximum number of times of fractionating, and bacteria are diluted with the culture medium to reduce the bacteria concentration, so that there is room for improvement in the detection sensitivity of bacteria.

The above problem is not limited to the bacteria. The same applies to detection of cells in general for highly sensitive and rapid detection without destroying a small number of cells such as cultured cells.

Therefore, the present disclosure provides a technique for detecting a cell with high sensitivity without destroying the cell.

### Solution to Problem

In order to solve the above problems, a cell detection device of the present disclosure includes: a medium adding device configured to add a part of a prepared culture medium to a culture container holding cells to be detected; a medium collection device configured to collect the culture medium from the culture container; and a detection device configured to detect light from the luminescent reagent mixed with the collected culture medium.

Other features of the disclosure will be clear from the description and the accompanying drawings. In addition, embodiments of the present disclosure are achieved and realized by elements, combinations of various elements, the following detailed description, and the attached claims.

The description of this specification is given only as a typical example, and does not limit the scope of claims or applications of the present disclosure.

### Advantageous Effects of Invention

According to the cell detection device of the present disclosure, it is possible to detect a cell with high sensitivity without destroying the cell.

Objects, configurations, and effects besides the above description will be apparent through the explanation on the following embodiments.

### Brief Description of Drawings

[FIG. 1] FIG. 1 is a schematic cross-sectional view illustrating a cell detection device according to a first embodiment.
[FIG. 2] FIG. 2 is a functional block diagram of the cell detection device according to the first embodiment.
[FIG. 3] FIG. 3 is a flowchart illustrating the cell detection method according to the first embodiment.
[FIG. 4A] FIG. 4A is a schematic view illustrating a measurement screen.
[FIG. 4B] FIG. 4B is a schematic view illustrating a setting screen of the measurement method.
[FIG. 4C] FIG. 4C is a schematic view illustrating a screen displaying a graph of a measurement result.
[FIG. 5] FIG. 5 is a schematic cross-sectional view illustrating a cell detection device according to the second embodiment.
[FIG. 6] FIG. 6 is a functional block diagram of the cell detection device according to the second embodiment.
[FIG. 7] FIG. 7 is a graph illustrating a result of detecting bacteria with the cell detection device according to the second embodiment.
[FIG. 8] FIG. 8 is a schematic view illustrating a cell detection device according to a third embodiment.
[FIG. 9] FIG. 9 is a schematic cross-sectional view illustrating a cell detection device according to a fourth embodiment.
[FIG. 10] FIG. 10 is a schematic view illustrating a configuration of a part of a cell detection device according to a fifth embodiment.
[FIG. 11] FIG. 11 is a schematic cross-sectional view illustrating a configuration of a part of a cell detection device according to a sixth embodiment.
[FIG. 12] FIG. 12 is a schematic view illustrating a cell detection device according to a seventh embodiment.
[FIG. 13] FIG. 13 is a schematic cross-sectional view illustrating a configuration of a part of a cell detection device according to an eighth embodiment.
[FIG. 14] FIG. 14 is a schematic cross-sectional view illustrating a configuration of a part of a cell detection device according to a ninth embodiment.
[FIG. 15] FIG. 15 is a schematic view illustrating a configuration of a part of a cell detection device according to a tenth embodiment.

### Description of Embodiments

### [First Embodiment]

### <Configuration of Cell Detection Device>

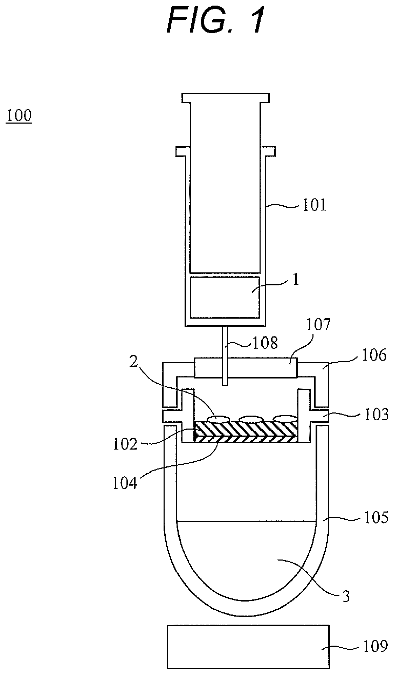

FIG. 1 is a schematic cross-sectional view illustrating a cell detection device 100 according to a first embodiment. The cell detection device 100 is a device for detecting bacteria or fungi (hereinafter, referred to as "bacteria") contained in a sample solution with high sensitivity by extracellular ATP secreted by the bacteria. As illustrated in FIG. 1, the cell detection device 100 includes a syringe 101, a filter holder 103 holding a filter 102, a collection container 105, a lid 106, and a luminescence measurement device 109.

The syringe 101 (medium container and medium adding device) accommodates a culture medium 1. The syringe 101 is configured to dispense a predetermined amount (part) of the total amount of the culture medium 1. Although not illustrated, the syringe 101 is attached to a drive device (medium adding device) that controls the movement of the syringe 101 itself in the vertical direction and the sliding of the plunger of the syringe 101.

A mesh 104 is provided at a bottom of the filter holder 103 (culture container). The filter 102 is disposed on the mesh 104. The bacteria 2 are captured by the filter 102 by filtering the sample solution using, for example, the filter 102 and a filtration device (not illustrated). The filter 102 is disposed on the mesh 104 of the filter holder 103 after filtering the sample solution for capturing the bacteria 2.

The pore diameter of the filter 102 is selected to have a size capable of capturing the bacteria 2 (cells) to be detected. For example, in the case of detecting bacteria, a filter having a pore diameter of 0.5 µm or less is generally used. In particular, in the case of detecting small bacteria, for example, a filter having a pore diameter of 0.2 um or 0.1 um is used. When an object having a large cell such as yeast or mold is to be detected, for example, a filter having a pore diameter of 1 um or the like can be used.

The shape of the filter 102 may be a generally commercially available circular shape or other shapes. For example, by using a tubular or roll-shaped filter, the diameter can be reduced while maintaining the filter area. When a plurality of filters are used side by side, more filters can be arranged in the same space as compared with a circular filter. In addition, even in the case of a circular shape, by forming wave-shaped irregularities instead of forming a flat surface of the filter, it is possible to increase the area of the filter and to prevent clogging of the filter due to particles contained in the sample. Further, also by using a filter having a large pore diameter with the filter having a small pore diameter stacked thereunder, it is possible to prevent clogging of the filter due to particles contained in the sample.

The pore diameter of the mesh 104 may be any size as long as the culture medium 1 can pass therethrough according to an operation of collecting the culture medium 1 by a medium collection device described later.

The lid 106 is configured to be detachable from the filter holder 103 to cover the space on the filter 102. As a result, contamination from the outside can be prevented. A septum 107 is provided on the upper surface of the lid 106. A predetermined amount of the culture medium 1 can be supplied onto the filter 102 while keeping the space on the filter 102 sealed by causing a needle 108 of the syringe 101 to penetrate the septum 107. The bacteria 2 captured and concentrated from the sample can be cultured on the filter 102 by supplying the culture medium 1 to the filter 102 obtained by filtering the sample solution.

In the example illustrated in FIG. 1, the filter holder 103 is formed in a cylindrical shape having a flange portion on a side surface. The lid 106 is supported on an upper surface of the flange portion. A bottom surface of the flange portion is supported by the collection container 105. The outer diameter of the filter holder 103 can be smaller than the inner diameter of the collection container 105. The outer diameter of the flange portion of the filter holder 103 can be substantially equal to the outer diameter of the collection container 105.

The collection container 105 is, for example, a container such as a microtube. The collection container 105 accommodates a luminescent reagent 3 containing, for example, luciferin and luciferase. After the bacteria 2 are cultured on the filter 102, for example, a centrifugal operation is performed by a centrifuge (medium collection device) in a state where the filter holder 103 and the collection container 105 are connected, whereby the culture medium 1 containing ATP, which is a secretion of the bacteria 2, is collected in the collection container 105. As a result, ATP in the culture medium 1 collected in the collection container 105 and the luminescent reagent 3 are mixed, and luminescence by the reaction can be generated. As long as the culture medium 1 on the filter 102 can be collected in the collection container 105, a medium collection device other than a centrifuge can also be used. For example, the culture medium 1 may be collected from the filter 102 to the collection container 105 by applying pressure such as air pressure from the side of the lid 106 using a pressure pump or the like.

The luminescence measurement device 109 (detection device) is disposed at a position where luminescence from the luminescent reagent 3 in the collection container 105 can be detected (for example, near the bottom of the collection container 105). The luminescence measurement device 109 outputs the detection signal to, for example, an external computing device. The computing device calculates the luminescence amount from the detection signal of the luminescence measurement device 109 and calculates the amount of ATP corresponding to the luminescence amount.

Although not illustrated, the cell detection device 100 may include a temperature controller for externally controlling at least the temperature of the filter 102.

The drive device of the syringe 101, the luminescence measurement device 109, the temperature controller, and the centrifuge are connected to the above-described computing device, and the computing device is connected to an input/output device such as a monitor, a keyboard, and a touch panel. The user can set various parameters by operating the input/output device, and the computing device operates the drive device, the luminescence measurement device 109, the temperature controller, and the centrifuge according to the parameters.

As illustrated in FIG. 1, in the cell detection device 100, a storage place (medium container) of the culture medium 1, the filter 102 (culture container) where the bacteria 2 are cultured, and a storage place (collection container) of the luminescent reagent 3 are disposed separately. The culture medium 1 moves in the order of the medium container, the culture container and the collection container. Further, it is notable that the culture medium 1 can be added to the filter 102 by a fixed amount instead of the entire amount using the syringe 101 (medium adding device) . With this configuration, it is possible to repeat operations of adding a small amount of the culture medium 1 from the syringe 101 to the filter 102, culturing the bacteria 2 on the filter 102, collecting the culture medium 1 to the collection container 105 by a centrifuge (medium collection device) to react with the luminescent reagent 3, and measuring bacteria-derived ATP in the culture medium 1. The amount of the culture medium 1 added to the filter 102 at one time will be described later.

By culturing the bacteria 2 in a small amount of the culture medium 1 on the filter 102, it is possible to increase the concentration of ATP secreted by the bacteria 2 in the culture medium 1 and thus to measure extracellular ATP with high sensitivity. As a result, bacteria can be quickly detected. In addition, since the extracellular secretion is measured, it is not necessary to destroy the bacteria 2 and it is possible to measure a temporal change accompanying the proliferation of the same bacteria sample. After the detection of the bacteria, the bacteria can be further subjected to enrichment culture and subjected to other tests (drug sensitivity test, bacterial species identification test, genetic test, etc.), or the bacterial strain can be preserved.

In the cell detection device 100, the luminescent reagent 3 is stored in advance in the collection container 105. The culture medium 1 collected from the filter 102 is repeatedly mixed in the collection container 105. In this way, since a mechanism for dispensing the luminescent reagent 3 is not required, the device cost can be suppressed.

When a purpose is to detect anaerobic bacteria, the collection container 105, the filter holder 103, and the lid 106 are brought into close contact with each other to maintain airtightness of the inside. The inside is filled with a gas containing no oxygen. The culture medium 1 is degassed in advance and filled in the syringe 101. Thus, anaerobic culture can be performed. A reducing agent may be added to the culture medium 1 to produce an anaerobic environment. On the other hand, since the luminescent reagent in the ATP method requires oxygen, oxygen may be enclosed in the space on the luminescent reagent 3 side.

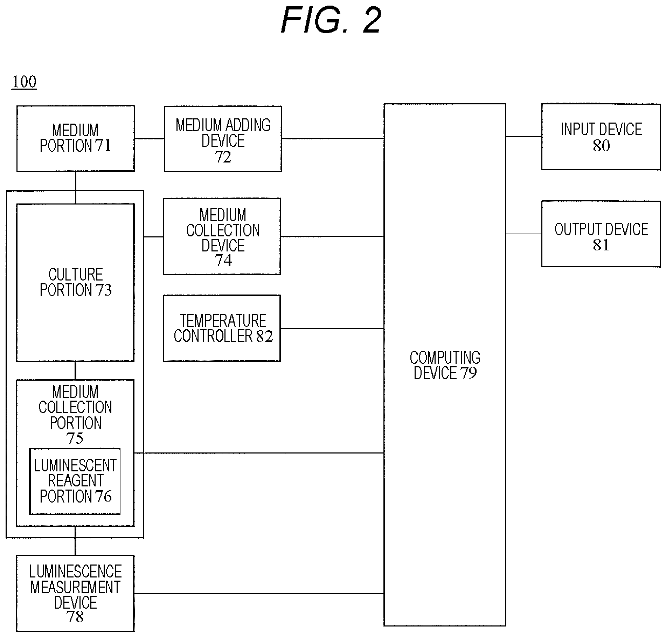

FIG. 2 is a functional block diagram of the cell detection device 100 according to the first embodiment. As illustrated in FIG. 2, the cell detection device 100 includes a medium portion 71 (medium container), a medium adding device 72, a culture portion 73 (culture container), a medium collection device 74, a medium collection portion 75 (collection container), a luminescent reagent portion 76 (collection container), a luminescence measurement device 78 (detection device), a computing device 79, an input device 80, an output device 81, and a temperature controller 82.

The medium portion 71 is a place where a culture medium suitable for the cell to be detected is accommodated. The medium portion 71 is, for example, a storage portion and so on of the culture medium 1 of the syringe 101 illustrated in FIG. 1. The medium adding device 72 is connected to the medium portion 71. The medium adding device 72 is, for example, a syringe 101 and a drive device thereof. The drive device includes a component for fixing the syringe 101, a motor for moving the component, a motor for pressing a plunger of the syringe 101 by a certain distance to discharge the culture medium 1, and the like.

Bacteria (cells) to be detected are held in the culture portion 73. The culture portion 73 corresponds to, for example, the filter 102 and the filter holder 103 of the cell detection device 100. A part of the culture medium is supplied from the medium portion 71 to the culture portion 73. In the cell detection device 100, the culture medium held in the medium portion 71 is separated from the culture medium supplied from the medium portion 71 to the culture portion 73 by accommodating them in different containers. These containers are configured so that the culture medium and components in the culture medium do not move from the culture portion 73 to the medium portion 71.

The medium collection device 74 performs an operation of moving the culture medium added to the culture portion 73 to the culture medium collection portion 75. For example, the medium collection device 74 includes a device for conveying liquid, such as a centrifuge, a suction pump, a pressure pump, or a tube pump.

The culture medium collection portion 75 accommodates the culture medium transferred from the culture portion 73. The medium collection portion 75 corresponds to, for example, the collection container 105 and so on illustrated in FIG. 1. In the cell detection device 100, a culture medium collecting operation by the medium collection portion 75 is performed in a state where the culture portion 73 and the medium collection portion 75 are connected.

The luminescent reagent portion 76 is a place for holding a luminescent reagent for a luminescent reaction. The luminescent reagent portion 76 corresponds to, for example, a container or syringe containing a luminescent reagent and so on. In the cell detection device 100, since the luminescent reagent 3 is stored in the collection container 105, the luminescent reagent portion 76 and the culture medium collection portion 75 are integrated.

The luminescence measurement device 78 is a device that detects light generated by a luminescence reaction in the medium collection portion 75. Specifically, the luminescence measurement device 78 includes a sensor such as a CCD sensor, a CMOS sensor, or a photomultiplier tube. The luminescence measurement device 78 outputs a detection signal of luminescence from the luminescent reagent portion 76 to the computing device 79.

The computing device 79 is, for example, a computer terminal such as a personal computer, a smartphone, a tablet, or a mobile phone. The computing device 79 executes processing of a detection signal from the luminescence measurement device 78. Specifically, when receiving the input of the detection signal from the luminescence measurement device 78, the computing device 79 calculates the luminescence amount from the detection signal and calculates the amount of ATP according to the luminescence amount.

In addition, the medium adding device 72 and the medium collection device 74 are connected to the computing device 79. The computing device 79 controls operations of the medium adding device 72 and the medium collection device 74. The input device 80 and the output device 81 are connected to the computing device 79. A user can determine a measurement parameter via the input device 80. The computing device 79 controls operations of the medium adding device 72 and the medium collection device 74 according to the parameter. The output device 81 displays a calculation result of the amount of ATP by the computing device 79, a GUI screen for the user to input the measurement parameter, and the like.

The temperature controller 82 corresponds to, for example, an incubator, and adjusts a temperature of the culture portion 73 to an appropriate temperature according to the type of bacteria.

As described above, the medium portion 71, the culture portion 73, and the medium collection portion 75 are separately disposed, and luminescence measurement is performed in the medium collection portion 75. Thus, it is possible to repeat operations of intermittently adding a predetermined amount of the culture medium from the medium portion 71 to the culture portion 73 and collecting the culture medium to the medium collection portion 75 after culture, and it is also possible to measure bacteria nondestructively and with high sensitivity.

### <Cell Detection Method>

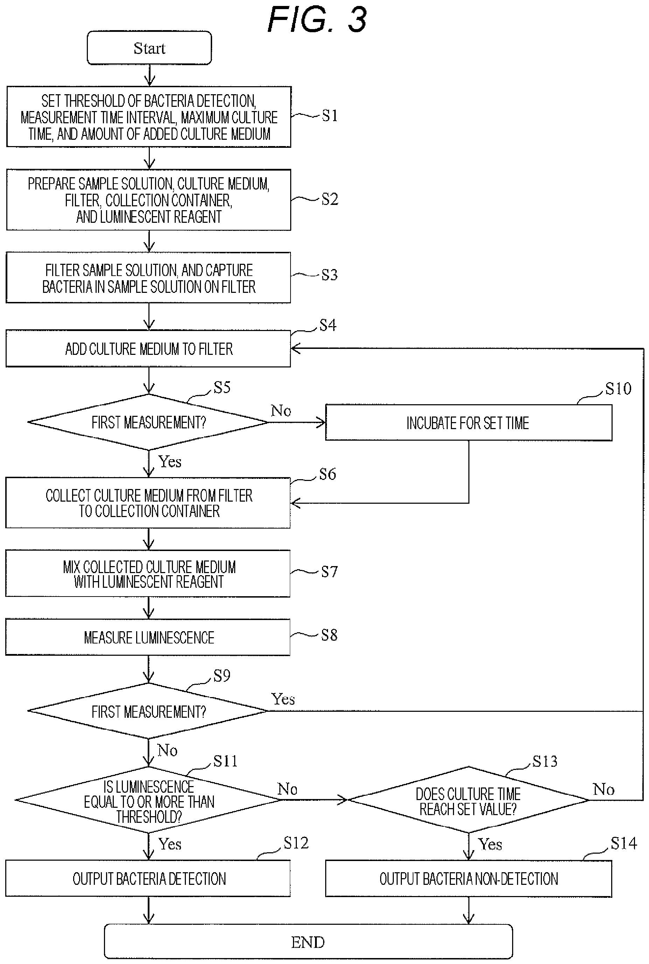

FIG. 3 is a flowchart illustrating a cell detection method using the cell detection device 100.

In Step S1, the user sets the threshold for bacteria detection, the culture time (measurement time interval), the maximum culture time, and the medium addition amount in one culture by operating the GUI screen for condition setting displayed on the output device 81 and the input device 80. The computing device 79 stores these set parameters in a storage device (not illustrated in FIGS. 1 and 2).

In Step S2, the user prepares a certain sample solution possibly containing bacteria, the syringe 101 for accommodating the culture medium 1, the filter 102, the filter holder 103, the lid 106, the collection container 105, and the luminescent reagent 3.

In Step S3, the user connects the filter holder 103 in which the filter 102 is set to a filtration device (not illustrated). Next, the user connects a funnel (not illustrated) to the bacteria capturing surface side of the filter 102 to pour the sample solution into the funnel for filtration. As a result, the bacteria 2 are captured and concentrated on the filter 102, and the solvent is removed. It is possible to adopt a method suitable for the sample, as the filtration by the filter 102, such as suction filtration, pressure filtration, or centrifugal filtration. After the filtration, a washing liquid that does not affect bacteria is further added and filtered, so that the filter 102 can be washed to reduce the luminescence measurement background derived from the sample solution.

Next, the user introduces the luminescent reagent 3 into the collection container 105. Then, the user connects the filter holder 103 to the upper portion of the collection container 105. Thereafter, the user puts the lid 106 on the filter holder 103 to prevent bacteria from entering the filter 102 from the outside world.

When Steps S1 to S3 are completed, the user inputs an operation start instruction to the computing device 79 by operating the input device 80.

In Step S4, the computing device 79 drives the medium adding device 72 based on the medium addition amount set in Step S1 to cause the septum 107 of the lid 106 to penetrate the needle 108 of the syringe 101. The computing device 79 adds a part of the culture medium 1 from the syringe 101 to the filter 102. The amount of the culture medium 1 to be added can be the minimum amount necessary to wet the entire filter 102.

In Step S5, the computing device 79 determines whether the measurement to be executed is the first luminescence measurement. In the case of the first measurement (Yes), the process proceeds to Step S6.

In Step S6, the computing device 79 drives the medium adding device 72 to pull out the needle 108 of the syringe 101 from the septum 107. Thereafter, in a state where the lid 106, the filter holder 103, and the collection container 105 are connected, the computing device 79 moves them to the medium collection device 74 to collect the culture medium in the filter holder 103 (on the filter 102) into the collection container 105. When the medium collection device 74 is a centrifuge, the culture medium held by the filter 102 can be dropped toward the collection container 105 by a centrifugal force. By the collection operation in Step S6, the collected culture medium and the luminescent reagent 3 are mixed (Step S7) .

In Step S8, the luminescence measurement device 109 detects luminescence generated by the reaction between ATP in the culture medium and the luminescent reagent 3 to output a detection signal to the computing device 79. The computing device 79 calculates the luminescence amount based on the detection signal. Then, the computing device 79 stores the calculated amount in the storage device as the luminescence amount at the 0 hour of culture.

In Step S9, the computing device 79 determines whether the measurement is the first measurement. In the case of the first measurement, the process returns to Step S4. In Step S4, the computing device 79 adds the same amount of the culture medium 1 as that in the previous Step S4 onto the filter 102. Thereafter, in Step S5, the computing device 79 determines that the measurement is not the first measurement (No), and thus, the process proceeds to Step S10.

In Step S10, the computing device 79 cultures bacteria on the filter 102 for a predetermined time while maintaining the temperature of the filter 102 at an appropriate temperature by the temperature controller 82. The temperature may be set according to bacteria to be detected, such as about 30 to 40°C for bacteria and about 20 to 30°C for mold and so on.

Thereafter, Steps S6 to S9 are executed in the same manner as described above. When culturing for 1 hour is performed, the computing device 79 stores the luminescence amount calculated in the second Step S8 in the storage device as the luminescence amount for 1 hour after culture.

In Step S11, the computing device 79 determines whether the luminescence amount after culture is equal to or greater than the threshold set in Step S1. When it is equal to or more than the threshold (Yes), since the luminescence amount is increased by ATP being secreted into the culture medium as the bacteria proliferates, the computing device 79 determines that the bacteria are detected (positive) in Step S12 to output the determination result to the output device 81. When the output device 81 is a monitor, a screen indicating a positive result is displayed. When the output device 81 is a speaker, an alarm sound or the like is emitted.

When the luminescence amount after culture is less than the threshold in Step S11 (No), the process proceeds to Step S13. In Step S13, the computing device 79 determines whether the culture time has reached the maximum culture time set in Step S1. When the maximum culture time has been reached (Yes), in Step S14, the computing device 79 determines that the bacteria are not detected (negative) to output the determination result to the output device 81.

When the maximum culture time has not been reached in Step S13 (No), the process returns to Step S4. So, the addition of the culture medium, the culture, the collection container of the culture medium, and the luminescence measurement are repeated again.

As described above, when the luminescence amount is equal to or greater than the threshold and it is determined as positive, or when the maximum culture time is reached and it is determined as negative, the detection of bacteria is terminated. In the determination in Step S13, it may be determined whether the maximum number of times of measurement has been reached.

For the determination of the increase in the luminescence amount in Step S11, the threshold set by the user in Step S1 may be used as described above, or the computing device 79 may automatically calculate from the measurement data. In addition, the user may be allowed to make settings on the GUI screen for condition setting displayed on the output device 81 at the stage of Step 511. As a result, even in the detection target having different characteristics, it is possible to appropriately set the detection condition and to perform inspection by the same cell detection device 100 and cell detection method.

In addition, the determination of the increase in the luminescence amount in Step S11 is not limited to the method based on the comparison between the measured luminescence amount and the threshold as described above. For example, a difference between the measured luminescence amount and the luminescence amount at 0 hour, or a difference between the luminescence amount measured this time and the previous luminescence amount may be compared with their thresholds.

The allowable range of the culture time set in Step S1 is determined from the proliferation rate or growth rate of bacteria, the amount of culture medium prepared at the beginning, the amount of luminescent reagent, and the like. The amount of ATP secreted outside bacteria is considered to be proportional to the number of bacteria. In general, the number of bacteria is doubled in about 20 minutes in the bacteria species (E. coli and the like) that divide the fastest. Therefore, in order to detect the proliferation of bacteria by increasing extracellular ATP, it is considered appropriate that the incubation time is 20 minutes or more, so that the measurement interval can be longer than 20 minutes in consideration of measurement variations and measurement sensitivity. For example, the time interval is one hour or two hours.

For the amount of the culture medium and the amount of the luminescent reagent prepared in Step S2, the required amounts are calculated from the amount of the culture medium added at one time and the set value of the maximum number of times of measurement. For example, when the medium addition amount to the filter is 50 µL and it is desired to set the maximum number of times of the culture medium addition to 20 times, the amount of the culture medium in the syringe is required to be 1 mL. Considering that the luminescent efficiency decreases in proportion to the thinning concentration of the luminescent reagent every time the culture medium is mixed with the luminescent reagent, for example, when the dilution rate of the luminescent reagent is allowed up to 1.5 times dilution, assuming that the amount of the culture medium collected from the filter is 50 µL each time and the maximum number of times of measurement is 20 times, 2 mL of the luminescent reagent stored in advance in the collection container is required. It is possible to determine the allowable dilution rate of the luminescent reagent by measuring the relationship between the dilution rate and the luminescence intensity in advance using a standard ATP solution.

The type of culture medium to be added can be selected according to the bacteria to be detected. In general bacteria, a broth medium, a soybean-casein digest medium, or the like can be used, but not limited thereto. A culture medium selected for the purpose of detecting only specific bacteria can also be used. For example, when it is intended to detect a group of Enterobacteriaceae bacteria, an EE broth medium for detecting a group of Enterobacteriaceae bacteria can be used.

By reducing the amount of the culture medium to be added onto the filter 102 as much as possible in Step S4, the extracellular ATP concentration can be increased. On the other hand, it is necessary to add the culture medium uniformly distributed over the entire filter or an amount of a sufficient nutrient to the bacteria captured on the filter or more. It is also necessary to consider the amount of water evaporated during incubation.

As an example, the amount of the added culture medium was examined using a PVDF filter having an area of 0.2 cm², a thickness of 0.125 mm, and a pore diameter of 0.45 um. When the volume of this filter is calculated as area multiplied by thickness, it is 25 µL. When 50 µL of the culture medium was added to the filter to sufficiently wet the entire filter and then the filter was centrifuged by the centrifuge to remove the culture medium, 5 µL of the culture medium remained on the filter. In addition, when, after adding 50 µL of the culture medium to the filter, the filter was placed in a sealed container and then left in an incubator at 37°C for 2 hours, moisture in the culture medium on the filter evaporated by about 10 µL. Therefore, the amount of the culture medium to be added to the filter can be 20 µL obtained by adding the minimum amount required for the luminescence reaction, for example, 5 µL, to 15 µL obtained by adding the residual content and the evaporation content. More preferably, when the filter volume is adjusted to 40 µL or more by adding the residual content and the evaporation content to the filter volume, it is considered possible to maintain the state in which the culture medium spreads over the entire filter even if there is evaporation and thus to reduce damage to bacteria.

As described above, it is considered that the amount of the culture medium to be added to the filter is appropriately about 1/2 times or more and 5 times or less, or about 1 to 2 times the area multiplied by thickness of the filter.

As the substance to be measured, various secretions can be used in addition to ATP secreted from bacteria to the outside of bacteria. In addition to the secretion, it is also possible to detect bacteria by adding a substrate that generates fluorescence or luminescence by being decomposed by a target enzyme for a specific enzyme possessed by the bacteria.

For example, it is conceivable to add a luciferin derivative that is decomposed by a bacterial esterase to produce luciferin to the culture medium 1. The luciferin derivative is decomposed in proportion to esterase activity of bacteria, and luciferin is generated in the culture medium 1. When a reagent containing ATP and luciferase is added thereto as a luminescent reagent, released luciferin can be quantified. By this method, bacterial proliferation can be detected based on esterase activity.

In addition, by using a luciferin derivative that is decomposed only by an enzyme specific to a bacterial species to produce luciferin, it is also possible to detect only a specific bacterial species. Examples thereof include a D-luciferin-O-β-D-glucuronide derivative decomposed by β-glucuronidase specific to E. coli, and a luciferin derivative 6-O-β-Galactopyranosyl-Luciferin for β-galactosidase specific to E. coli group.

Here, the quantification by the bioluminescence method using luciferin has been described, but it is also possible to detect fluorescence using a derivative of a fluorescent substance. In addition, it is also possible to simultaneously detect different enzyme activities using a plurality of luminescent substances or derivatives of fluorescent substances having different wavelengths. In the case of detecting fluorescence, an excitation light source and a light measurement device are used instead of the luminescence measurement device 78.

In addition, the technology of the present embodiment can be applied not only to bacteria but also to a method for detecting the activity or proliferation of cultured animal cells or the like. The proliferation of the target cell can be detected by adding a substrate corresponding to various enzymes specific to the target cell.

### <Example of GUI Screen>

An example of a GUI screen displayed on the output device 81 when bacteria are detected for a plurality of sample solutions will be described.

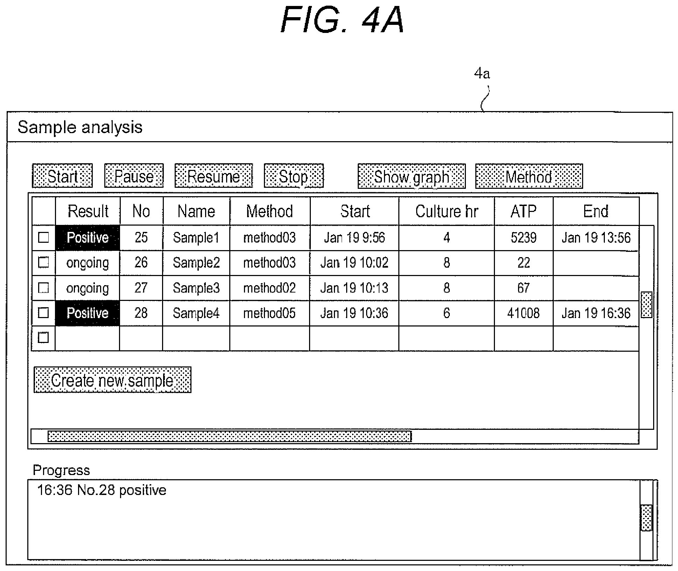

FIG. 4A is a schematic view of a measurement screen 4a illustrating an inspection condition and an inspection status of a plurality of sample solutions. As illustrated in FIG. 4A, a new line is created by clicking the "Create new sample" button on the measurement screen 4a. It is possible to set the measurement of the new sample by inputting the number, name, and other information of the sample are input on the measurement screen 4a. The measurement can be started by clicking the "Start" button. The measurement can be stopped by clicking the "Pause" button. In addition, displayed is the culture start time, the culture elapsed time, the positive or negative detection, or the ongoing of measurement for each sample. The end time is displayed for the sample from which the inspection result has been obtained. If the most recent ATP measurement is outside the settable range, an error is displayed.

When the user clicks the "Method" button on the measurement screen 4a, the measurement method setting screen opens so that the measurement method can be set.

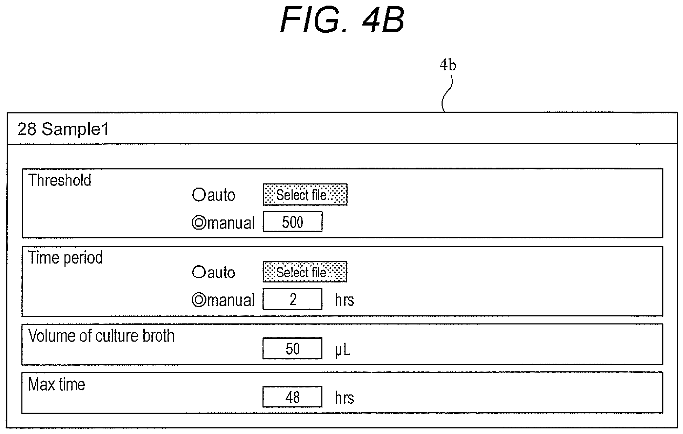

FIG. 4B is a schematic view illustrating a measurement method setting screen 4b. As illustrated in FIG. 4B, the measurement method setting screen 4b can be called to set the measurement method for one or more samples selected on the measurement screen 4a. The measurement method setting screen 4b is provided with a field in which it is possible to set a threshold as a criterion for determining positive bacteria, a time interval of measurement (culture time), the amount of culture medium added from the medium portion to the culture portion, maximum value of the culture time (test time), and the like. It is also possible to set a calculation method of a threshold or a time interval in the computing device 79 in advance to call and use the calculation method.

When the user clicks a "Show graph" button on the measurement screen 4a of FIG. 4A, the graph screen can be called up for one or more samples selected on the measurement screen 4a.

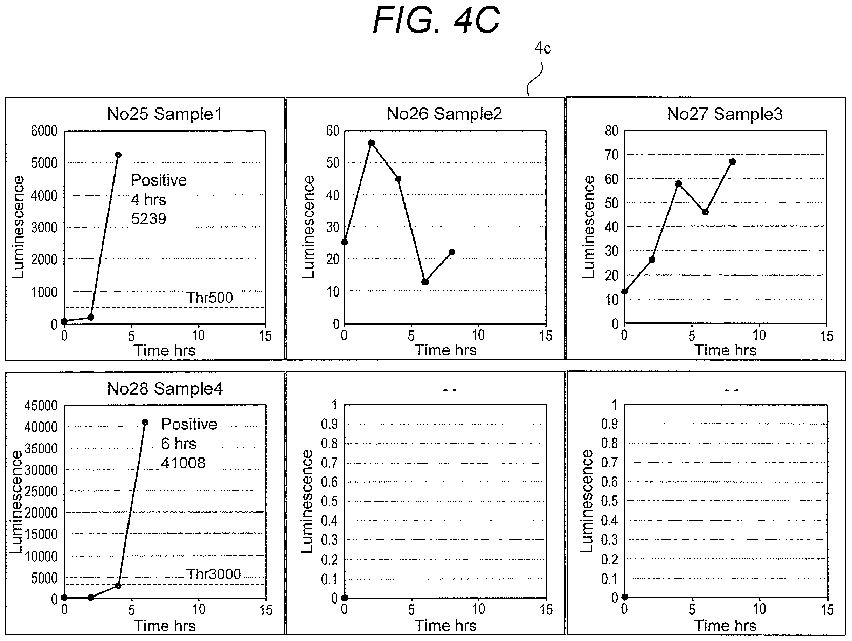

FIG. 4C is a schematic view illustrating a graph screen 4c. As illustrated in FIG. 4C, the graph screen 4c displays, for the selected specimen, a temporal change of the measured value, a determination time and a result when positive/negative determination is made, a threshold used for the determination, and the like. By using the graph screen 4c, it is possible to visually confirm the progress of measurement for a plurality of samples.

### <Technical advantage>

As described above, the cell detection device 100 according to the first embodiment includes the syringe (medium container and medium adding device) that accommodates the culture medium, the filter 102 (culture container) that holds the bacteria 2, the medium collection device such as a centrifuge, and the collection container 105 that accommodates the luminescent reagent 3. The cell detection device 100 adds a part of the culture medium 1 in the syringe 101 to the filter 102 by the syringe 101. The cell detection device 100 collects the culture medium 1 in contact with the bacteria 2 in the collection container 105 by the medium collection device to measure luminescence from the luminescent reagent 3 by the luminescence measurement device 109. As described above, since the culture medium in contact with the bacteria is collected from the filter 102, it is possible to culture the bacteria 2 by adding a part of the culture medium 1 to the filter 102 holding the bacteria 2 again. Therefore, the cell detection device 100 can repeat a series of operations of contact between a part of the culture medium 1 and the bacteria 2, movement of the culture medium 1 in contact with the bacteria 2, and detection of light a plurality of times for each culture time.

With such a configuration, the cell detection device 100 can collect the extracellular secretion of the bacteria 2 captured on the filter 102 at a high concentration and measure luminescence in any culture time. Therefore, it is possible to quickly detect the presence or absence of proliferation of bacteria with high sensitivity without destroying the bacteria 2. In addition, since the detected bacteria 2 are non-destructive, it is possible to perform other inspection on the bacteria 2.

### [Second Embodiment]

In the first embodiment, a method has been described in which a cell detection device having one collection container is used to collect a culture medium after culture in the same collection container for each culture time and measure luminescence. On the other hand, in the second embodiment, a method is proposed in which a culture medium is collected in different collection containers for each culture time using a plurality of collection containers. In the present embodiment, the same components as those of the first embodiment are denoted by the same reference numerals, and repeated description is omitted.

### <Configuration of Cell Detection Device>

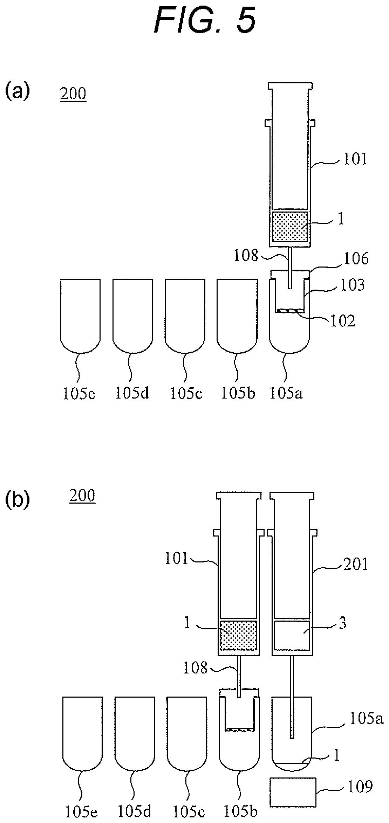

FIG. 5(a) is a schematic cross-sectional view illustrating a cell detection device 200 according to the second embodiment, and illustrates a state when the culture medium 1 is added to the filter 102 at the first time. As illustrated in FIG. 5(a), the cell detection device 200 includes five collection containers 105a to 105e (a plurality of collection containers). In addition, the luminescent reagent 3 is not introduced into the collection container 105 in advance. Other configurations are similar to those of the cell detection device 100 of the first embodiment. Note that the filter holder 103 and the lid 106 are illustrated in a simplified manner. The number of the collection containers 105 is 5 in FIG. 5 (a), but may be selected according to the number of times of luminescence measurement required.

The plurality of collection containers 105 are, for example, individual microtubes, and can be individually installed in a centrifuge, a luminescence measurement device, or a temperature controller. In the state illustrated in FIG. 5(a), the filter holder 103 having the filter 102 is connected to the first collection container 105a. The needle 108 of the syringe 101 is inserted into the septum of the lid 106 to add a part of the culture medium 1. Then the needle 108 of the syringe 101 is pulled out. Thereafter, in a state where the collection container 105a and the filter holder 103 are connected, the culture medium 1 on the filter 102 is set in a centrifuge and centrifuged, and collected in the collection container 105a.

FIG. 5(b) illustrates a state after the culture medium 1 is collected in the first collection container 105a. The syringe 101 and the filter holder 103 are retracted from the first collection container 105a from which the culture medium 1 has been collected to be set in the second collection container 105b. In addition, the luminescent reagent 3 is added to the first collection container 105a using a syringe 201 containing the luminescent reagent 3. Then luminescence measurement is performed by the luminescence measurement device 109. By moving either the luminescence measurement device 109 or the collection containers 105a to 105e, luminescence measurement can be performed for each collection container 105.

Although not illustrated, the syringe 201 is attached to a drive device (luminescent reagent adding device) that controls the movement of the syringe 201 itself in the vertical direction and the sliding of the plunger of the syringe 201.

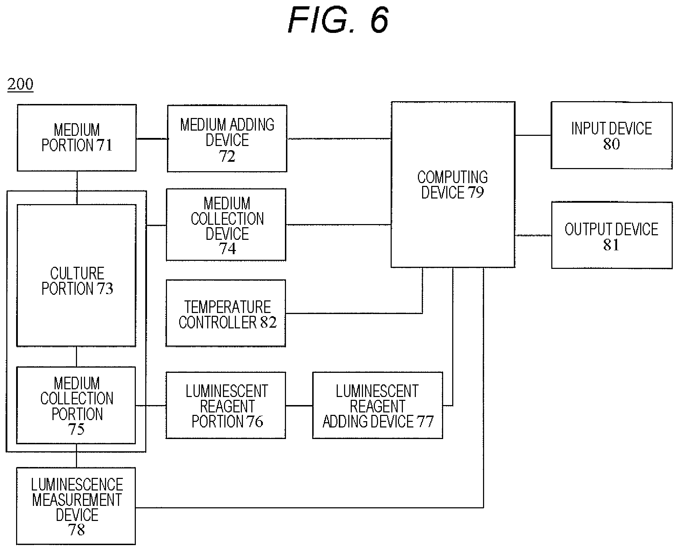

FIG. 6 is a functional block diagram of the cell detection device 200 according to the second embodiment. As illustrated in FIG. 6, in the cell detection device 200, the medium collection portion 75 and the luminescent reagent portion 76 are arranged separately. A luminescent reagent adding device 77 is connected to the luminescent reagent portion 76. The other points are similar to those of the cell detection device 100 of the first embodiment illustrated in FIG. 2.

In the second embodiment, the luminescent reagent portion 76 is, for example, a storage portion for the luminescent reagent 3 of the syringe 201 illustrated in FIG. 5(b). The luminescent reagent adding device 77 is, for example, the syringe 201 and a drive device thereof. The drive device of the syringe 201 includes a component for fixing the syringe 201, a motor for moving the component, a motor for pushing a plunger of the syringe 201 by a certain distance to discharge the luminescent reagent 3, and the like.

The computing device 79 controls the operation of the luminescent reagent adding device 77 in addition to the operation of the medium adding device 72 and the medium collection device 74. The user can determine the amount of the luminescent reagent 3 to be added via the input device 80. The computing device 79 controls the operation of the luminescent reagent adding device 77 according to the parameter.

### <Cell Detection Method>

Since the cell detection method using the cell detection device 200 according to the second embodiment is substantially similar to that of the first embodiment (FIG. 3), only differences from the first embodiment will be described below.

First, in the first measurement, Steps S1 to S6 are performed in the same manner as described above, and the culture medium 1 is collected in the first collection container 105a. After completion of Step S6, the computing device 79 retracts the filter holder 103 from the first collection container 105a by a mechanism (not illustrated) to connect these to the new second collection container 105b.

In Step S7, the computing device 79 drives the luminescent reagent adding device 77 to add the luminescent reagent 3 from the syringe 201 to the first collection container 105a.

Thereafter, Steps S8 and S9 are performed for the first collection container 105a in the same manner as in the first embodiment. The process returns to Step S4 The computing device 79 adds the culture medium from the syringe 101 to the filter 102 set in the second collection container 105b.

Next, in Step S5, it is determined that the measurement is the second measurement. Then, the process proceeds to Step S10, and culture is performed for a predetermined time.

Next, in Step S6, the culture medium 1 is collected in the second collection container 105b. After completion of Step S6, the computing device 79 retracts the filter holder 103 from the second collection container 105b by a mechanism (not illustrated) to connect these to the new third collection container 105c.

In Step S7, the computing device 79 drives the luminescent reagent adding device 77 to add the luminescent reagent 3 from the syringe 201 to the second collection container 105b.

Thereafter, for the second collection container 105b, Steps S8 to S14 are performed in the same manner as in the first embodiment. The above operation is repeated until a positive or negative detection result is obtained in the luminescence measurement after the second collection container 105b.

### <Technical Effect>

As described above, the cell detection device 200 according to the second embodiment includes a plurality of collection containers. The culture medium 1 after the bacteria are cultured on the filter 102 is collected in different collection containers for each repeated luminescence measurement. The collected culture medium 1 in each luminescence measurement is mixed with the luminescent reagent 3. As a result, since the mixing ratio of the collected culture medium 1 and the luminescent reagent 3 can be kept constant, luminescence measurement can be performed with the same sensitivity for any culture time. Therefore, the detection accuracy of bacteria is improved.

### <Experimental Example>

An experiment of detecting bacteria (cells) in a sample solution was performed using the cell detection device 200 according to the second embodiment.

### <<Preparation of Sample and Cell Detection Device>>

The detection target was set to Staphylococcus (S. aureus). Bacterial solution of Staphylococcus was used as a sample solution. Three types of bacterial numbers, 0 CFU (Colony forming unit), 10 CFU, or 100 CFU, were prepared.

The following components were used as components of the cell detection device 200.
Filter: Area of 0.2 cm², pore diameter of 0.45 µm
Collection container: Microtube
Culture medium: Soybean-casein digest medium (SCD medium)
Luminescent reagent: Luminescent reagent containing luciferin and luciferase

### <<Measurement of Amount of ATP>>

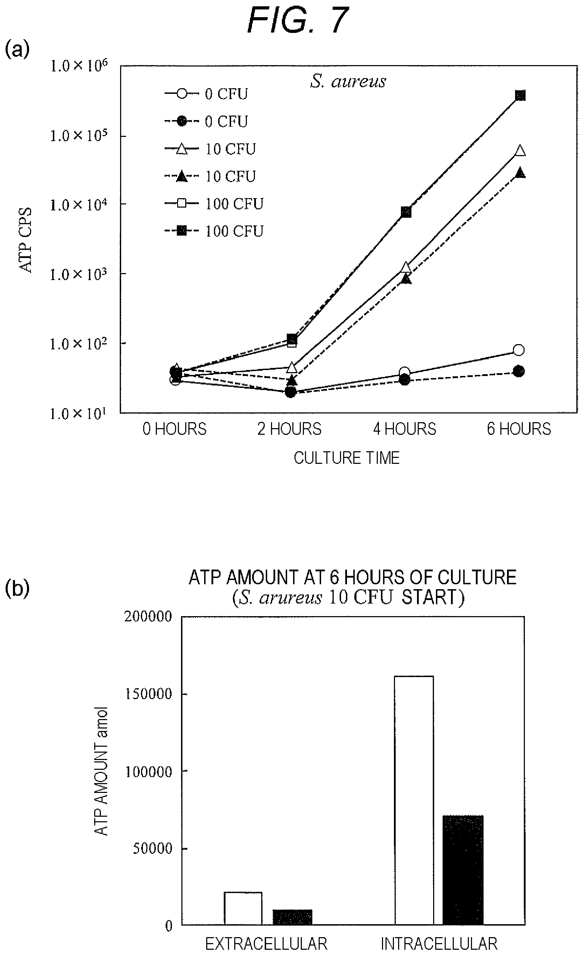

(1) First, a bacterial solution of Staphylococcus was added onto the filter, and centrifuged with a centrifuge in a state of being placed on a microtube and capped. As a result, bacteria were collected and concentrated on the filter, and the solvent was removed.
(2) Next, the microtube was replaced with a new one (first collection container). 50 µL of SCD culture medium was added to the filter. This was centrifuged Then, the culture medium added to the filter was collected in a microtube.
(3) 30 µL of a luminescent reagent was added to the collected culture medium to measure the luminescence amount. The unit of luminescence measurement is CPS (Count per second).
(4) Next, the filter was transferred to a new microtube (second collection container). Then, 50 µL of SCD medium was added to the filter again, and the filter was capped. This was incubated at 37°C for 2 hours. Then the culture medium was collected in a microtube (second collection container) by using a centrifuge. 30 µL of a luminescent reagent was added to the collected culture medium to measure the luminescence amount.
(5) Further, the operation of (4) was repeated twice to measure the luminescence amount after culture for 4 hours and the luminescence amount after culture for 6 hours.
(6) Two samples were prepared for each sample of 0 CFU, 10 CFU, or 100 CFU. The operations of (1) to (5) were performed for each sample. For each sample, the amount of ATP corresponding to the measured luminescence amount was calculated. The results are illustrated in FIG. 7 (a)

FIG. 7(a) is a graph illustrating a result of performing an inspection twice each for three types of sample solutions. The horizontal axis of the graph of FIG. 7(a) indicates the culture time. The vertical axis of the graph indicates the luminescence amount (CPS: Count per second) proportional to the amount of ATP. The ATP value at each time corresponds to the amount of extracellular ATP secreted into the culture medium during the 2 hour incubation. For example, the ATP value at 4 hours is the total amount of ATP secreted into the culture medium by the bacteria at 2 to 4 hours.

As illustrated in FIG. 7(a), no increase in ATP was observed between 0 hours and 6 hours for 0 CFU of Staphylococcus. At 10 CFU, no increase in ATP was observed at 2 hours, but an increase at 4 hours. At 100 CFU, an increase in ATP was observed at 2 hours.

### <<Comparison between Amount of Extracellular ATP and Amount of Intracellular ATP>>

After a sample of 10 CFU was cultured for 6 hours, intracellular ATP remaining in the filter was extracted by a known method to measure the amount of ATP.

FIG. 7(b) is a graph comparing the amount of extracellular ATP and the amount of intracellular ATP at 6 hours of culture. In FIG. 7(b), the vertical axis represents the amount of ATP (amol). The left two bar graphs are obtained by converting the unit of the measurement result of the amount of extracellular ATP at 10 CFU in FIG. 7(a) into amol. That is, Staphylococcus was cultured at an initial number of bacteria of 10 CFU. The amount of ATP secreted outside the bacteria during 4 to 6 hours was plotted.

Each of the two bar graphs on the right side shows the result of measuring the amount of intracellular ATP after measuring extracellular ATP using a sample of 10 CFU (measurement results of 2 times). That is, it shows the result of measuring the amount of ATP contained in the bacteria after culturing Staphylococcus at the initial number of bacteria of 10 CFU for 6 hours.

The amount of extracellular ATP was 15000 amol on average, and the amount of intracellular ATP was 120,000 amol. Therefore, it is found that the amount of extracellular ATP was about 1/8 of the amount of intracellular ATP.

### <<Comparison of Sensitivity of Cell Detection>>

Using 1 mL of a sample containing 10 CFU of Staphylococcus, the sensitivity in the case of measuring ATP by culturing for 6 hours according to the following three methods was calculated and compared using the above-described value of the amount of ATP.

### (Method 1) A case where a sample is added to a culture medium and cultured to measure intracellular ATP (bacterial destruction)

In this method, 9 mL of an SCD culture medium is added to 1 mL of a sample of 10 CFU/1 mL of Staphylococcus, and 30 µL of the SCD medium is fractionated for each while being cultured to measure intracellular ATP.

The initial bacterial concentration after addition of the SCD medium is 10 CFU/10 mL. This is cultured at 37°C for 6 hours, then 30 µL of the culture solution is fractionated. 10 µL of a free ATP erasing reagent and 10 µL of an intracellular ATP extraction reagent, and 30 µL of a luminescent reagent is added to the culture solution fractioned, to perform luminescence measurement. The amount of ATP is calculated to be 360 amol (120,000 amol ÷ 10 mL × 30 µL).

### (Method 2) A case where a sample is added to a culture medium and cultured, and extracellular ATP is measured (non-destructive bacteria)

When the bacteria are cultured in the same manner as in Method 1, 30 µL of a luminescent reagent is added to 50 µL of this culture solution to measure the luminescence amount. When the amount of ATP is calculated, the amount of ATP is 75 amol (15000 amol ÷ 10 mL × 50 µL).

### (Method 3) A case where bacteria are cultured in a small amount of culture medium on a filter (second embodiment)

1 mL of the sample is filtered through a filter. 10 CFU of bacteria are captured and concentrated on the filter. Then, 50 µL of the SCD culture medium is added and the mixture is cultured at 37°C for 2 hours. The culture medium is removed 3 times. When 30 µL of the luminescent reagent is added to 50 µL of the culture medium collected at the third time, luminescence is measured. When the amount of ATP is calculated, the amount of ATP is 15000 amol.

As described above, in the present embodiment, by culturing the bacteria concentrated on the filter in a small amount of culture medium and measuring the secretion outside the bacteria, it has been found that the sensitivity can be improved by about 50 times even if the bacteria are non-destructive as compared with a method in which a sample is fractionated from a normal culture solution every time and the amount of ATP is measured by luminescence. It has been also found that it is possible to detect the bacteria more quickly.

### [Third Embodiment]

In the second embodiment, the cell detection device having the plurality of individual collection containers has been described. On the other hand, in the third embodiment, a cell detection device having a plurality of collection containers integrally formed is proposed.

### <Configuration of Cell Detection Device>

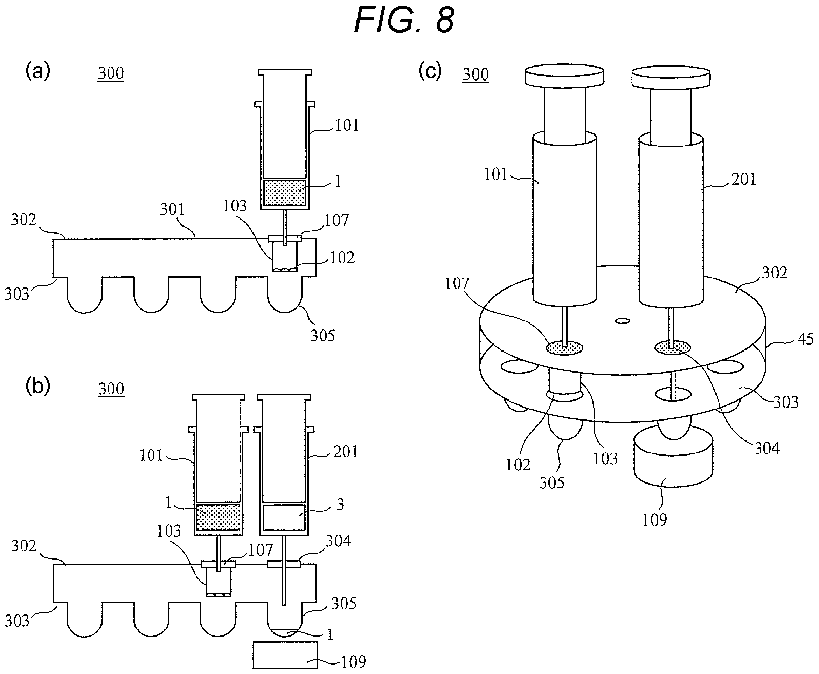

FIG. 8(a) is a schematic cross-sectional view illustrating a cell detection device 300 according to the third embodiment. As illustrated in FIG. 8, the cell detection device 300 includes a sealed container 301 whose inside is sealed. The sealed container 301 has a disk 302 forming an upper surface and a disk 303 forming a bottom surface.

One filter holder 103 (culture container) that holds the filter 102 is attached to the disk 302. An opening of the filter holder 103 is formed in the disk 302. The opening is closed by the septum 107.

A collection container 305 in the form of a plurality of wells is formed on the disk 303. The luminescent reagent 3 is not introduced into the collection container 305 in advance. At least one of the disk 302 or the disk 303 is configured to be rotatable about the center thereof as a rotation axis. Thus, the filter 102 can be located above each collection container 305. In order not to hinder the rotation of the disk 302 or 303, there is a gap between the opening of the collection container 305 and the filter 102 to such an extent that the culture medium does not leak out. The opening diameter of the collection container 305 is larger than the size of the filter 102. The rotation of the disk 302 or the disk 303 is controlled by, for example, a rotary electric machine that is driven based on an instruction of a computing device.

The space between the disks 302 and 303 is sealed. In the present specification, the term "sealed" means that there is no gap through which bacteria can enter from the outside. A gas having a composition different from that of the outside air can be enclosed in the sealed container 301 for anaerobic culture. Airtightness between the inside and the outside is maintained by the sealed structure.

FIG. 8(b) illustrates a state after the culture medium 1 is collected in the first collection container 305. By the rotation of the disk 302 or 303, the syringe 101 and the filter holder 103 are retracted from above the first collection container 305 from which the culture medium 1 has been collected. Then, the syringe 101 and the filter holder 103 are stopped above the second collection container 305. The disk 302 is provided with an opening covered with a septum 304. In addition, the luminescent reagent 3 is added to the first collection container 305 through the septum 304 with the needle of the syringe 201 using the syringe 201 storing the luminescent reagent 3 to perform luminescence measurement by the luminescence measurement device 109.

FIG. 8(c) is a perspective view illustrating the state of FIG. 8(b). In FIG. 8(c), the internal configuration is shown to be visible through the side wall surface of the sealed container 301. As illustrated in FIG. 8(c), the septum 107 and the septum 304 covering the opening provided in the disk 302 are located immediately above the two collection containers 305 provided in the disk 303. When the position of the luminescence measurement device 109 is fixed, the luminescence measurement can be performed using the plurality of collection containers 305 only by rotating the disk 302 by fixing the disk 303 and rotating the disk 303. Of course, the disk 303 may be fixed and the disk 302 may be rotated. The position of the luminescence measurement device 109 may be changed every time the luminescence is measured in each collection container 305.

### <Cell Detection Method>

Since the cell detection method using the cell detection device 300 according to the third embodiment is substantially similar to that of the second embodiment. Only differences from the second embodiment will be described below.

In the method of the present embodiment, in Step S3, the user sets the filter 102 capturing the bacteria 2 by filtering the sample solution in the filter holder 103 of the disk 302. Alternatively, the user may set the filter 102 on the disk 302, then add the sample solution to the filter 102, centrifuge the sealed container 301, and store the filtrate in one of the plurality of collection containers 305.

The method of the present embodiment is different from that of the second embodiment in that the positional relationship between the filter 102 and each collection container 305 is changed by rotating the disk 302 or 303.

### <Technical Effect>

As described above, in the cell detection device 300 according to the third embodiment, the filter 102 for culturing bacteria and the collection container 305 for measuring luminescence are disposed in the internal space of the sealed container 301. Then, the culturing of bacteria, the collection of the culture medium 1 and the measurement of luminescence are performed in the sealed container 301. As a result, it is possible to reduce the risk that bacteria are mixed to the collection container 305 from the outside when the filter 102 is moved to above different collection containers 305. In addition, since the gas composition in the sealed container 301 can be controlled, the culture conditions can be maintained at anaerobic or aerobic conditions depending on the type of bacteria to be detected.

### [Fourth Embodiment]

It is explained, in the second and third embodiments, that the method of adding a luminescent reagent to each of the plurality of collection containers by a syringe every time luminescence measurement is performed. On the other hand, proposed is, in the fourth embodiment, a cell detection device in which a luminescent reagent is dispensed in advance into a plurality of collection containers.

### <Configuration of Cell Detection Device>

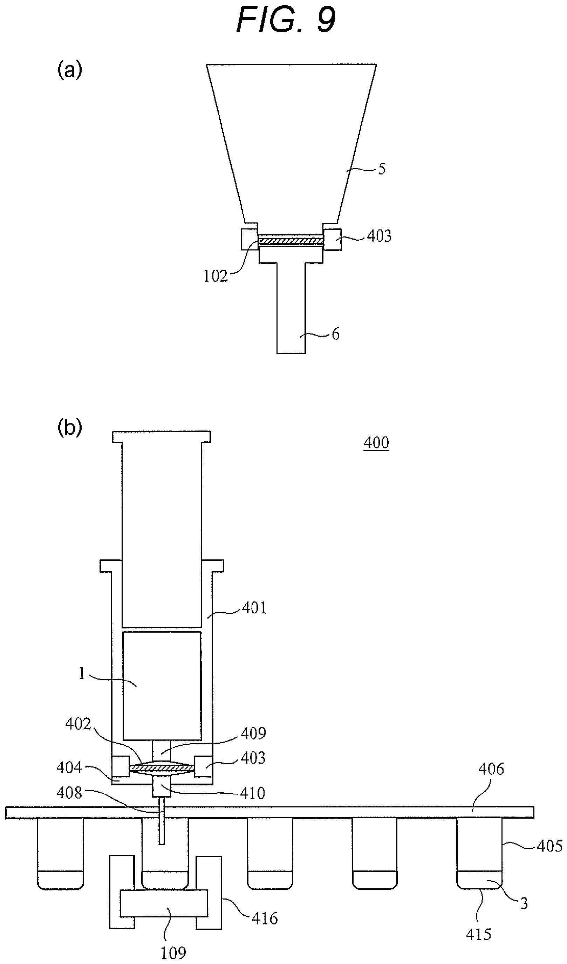

FIG. 9(a) is a schematic cross-sectional view for explaining a method for filtering a sample solution using the filter 102. The filter 102 is, for example, a filter having a volume of 100 µL. As illustrated in FIG. 9(a), the filter 102 is held by an annular filter holder 403. A large amount of liquid sample can be filtered by connecting a funnel 5 to the cell collection surface side of the filter 102 and by connecting a filtration stage 6 to the opposite surface. By connecting a suction pump (not illustrated) to the filtration stage 6 to perform suction filtration, bacteria in the sample can be collected and concentrated on the filter 102.

FIG. 9(b) is a schematic cross-sectional view illustrating a cell detection device 400 according to the fourth embodiment. As illustrated in FIG. 9(b), the cell detection device 400 is different from the cell detection device 100 to 300 of the first to third embodiments regarding the structures of a medium container that accommodates the culture medium 1 and a culture container in which culture is performed.

After bacteria are captured by the filter 102, the funnel 5 and the filtration stage 6 are removed from the filter holder 403. A syringe 401 (medium container and medium adding device) is connected to the cell collection surface side of the filter holder 403. An adapter 404 (member defining a space) is connected to the opposite surface. As a result, formed is a space 402 (culture container) surrounded by the bottom surface of the syringe 401, the inner wall surface of the filter holder 403 and the adapter 404. The filter 102 is accommodated in the space 402.

The syringe 401 is provided with a check valve 409 that controls dropping and sealing of the culture medium 1. The check valve 409 allows the culture medium 1 to flow only in the direction from the syringe 401 to the filter 102. During culture, the check valve 409 prevents diffusion of cell-derived substances from the filter 102 into the culture medium 1.

The adapter 404 is provided with a valve 410 and a capillary 408 for allowing to flow out the culture medium 1 that has passed through the filter 102. The valve 410 is configured to cause the culture medium to flow from the filter 102 to the capillary 408 only when a certain pressure or more is applied. The valve410 prevents the culture medium of the filter 102 from flowing toward the capillary 408 due to gravity while cells are cultured on the filter 102.

Although not illustrated, the syringe 401 is attached to a drive device (medium adding device) that controls movement of the syringe 401 itself in the vertical direction, sliding of the plunger of the syringe 401, driving of the check valve 409 and driving of the valve 410.

For example, 200 µL of the luminescent reagent 3 is dispensed into a plate-shaped container having a plurality of wells 405 (a plurality of collection containers). The well 405 is formed of a material that does not transmit light having a luminescence wavelength so that luminescence in the adjacent wells 405 does not interfere with measurement. A transparent window 415 is formed only in the vicinity of a surface on which luminescence measurement is performed by the luminescence measurement device 109. Each well 405 is covered with a seal 406 for maintaining sealing. As the seal 406, it is possible to use a rubber seal capable of maintaining the sealability even when the capillary 408 penetrates, an aluminum material that prevents deterioration of the luminescent reagent 3, or the like.

The luminescence measurement device 109 is provided with a cover 416 that covers the periphery of one well 405, so that it is possible to prevent light from the adjacent well 405 from being detected.

### <Cell Detection Method>

The cell detection method according to the fourth embodiment is substantially similar to the methods of the second and third embodiments using a plurality of collection containers. In the present method, the syringe 401 and the like are actually driven by the computing device driving each drive device. However, in order to simplify the description, the subject of the operation may be sometimes simply described as the computing device.

During Steps S3 and S4 described above with reference to FIG. 3, the computing device may operate the syringe 401 to flow the culture medium 1 and clean the flow path to the filter 102 and the capillary 408. At this time, the culture medium 1 discharged from the capillary 408 is discarded.

In the present embodiment, the addition amount of the culture medium 1 added onto the filter 102 at a time can be an amount (for example, 200 µL) obtained by adding the volumes of the space 402 where the filter 102 exists, the valve 410 and the capillary 408.

In the luminescence measurement at the time of culturing 0 hour, the computing device inserts the capillary 408 through the seal 406 into one well 405 to drop 200 µL of the culture medium 1 into the well 405 and to mix the culture medium 1 with the luminescent reagent 3. The luminescence measurement device 109 detects luminescence generated by the reaction through the transparent window 415 and transmits a detection signal to the computing device. The computing device calculates the luminescence amount corresponding to the detection signal to set the luminescence amount as luminescence at 0 hour.

Next, the computing device pulls out the capillary 408 from the well 405 in which luminescence measurement at time 0 has been performed The computing device maintains the filter 102 at, for example, 37°C by a temperature controller to culture bacteria on the filter 102.

After 2 hours of culture, the computing device causes the capillary 408 to penetrate the seal 406 and inserts the capillary into the well 405 different from the 0 hour. Then, 200 µL of the culture medium 1 is fed by the syringe 401 to drop into the well 405 the culture medium containing the substance secreted by the cells on the filter 102 and then to mix with the luminescent reagent 3. The luminescence measurement device 109 detects luminescence generated by the reaction through the transparent window 415 and transmits a detection signal to the computing device. The computing device calculates a luminescence amount corresponding to the detection signal to set the luminescence amount as luminescence at the second hour.

The computing device compares the luminescence amount at 0 hours with the luminescence amount at 2 hours to determine that bacteria are positive when a significant increase in the luminescence amount is observed at 2 hours. Otherwise, the same operation is repeated until an increase in the luminescence amount is detected as compared with the luminescence amount at 0 hours. As described above, the determination is not limited to the method of determining whether the measured luminescence amount is positive or negative by comparing the measured luminescence amount with the luminescence amount at 0 hours. The determination may be made by comparing the measured luminescence amount with a predetermined threshold determined in advance.

### <Technical Effect>

As described above, in the cell detection device 400 according to the fourth embodiment, the syringe 401 that accommodates and drops the culture medium 1 and the filter 102 that captures bacteria are used in combination. The culture medium 1 in the syringe 401 and the culture medium 1 added to the filter 102 are disposed separately. As a result, it is not necessary to separately move the syringe accommodating the culture medium and the filter holder 403 having the filter 102, and thus the operation is simple.

In addition, since the luminescent reagent 3 is dispensed to the plurality of wells 405 in advance, a dispensing mechanism of the luminescent reagent 3 is unnecessary. So, the configuration of the entire device can be simplified.

Further, as in the second embodiment, since the plurality of wells 405 into which a predetermined amount of the luminescent reagent 3 has been dispensed is used, the mixing ratio of the collected culture medium 1 and the luminescent reagent 3 can be kept constant as well as luminescence measurement can be performed with the same sensitivity for any culture time. Therefore, the detection accuracy of bacteria is improved.

### [Fifth Embodiment]

It has been explained in the first to fourth embodiments that the cell detection device is configured to add a culture medium in a direction substantially perpendicular to the filter surface. On the other hand, proposed is, in the fifth embodiment, a configuration in which a culture medium is added from a direction substantially horizontal to a filter surface.

### <Configuration of Cell Detection Device>

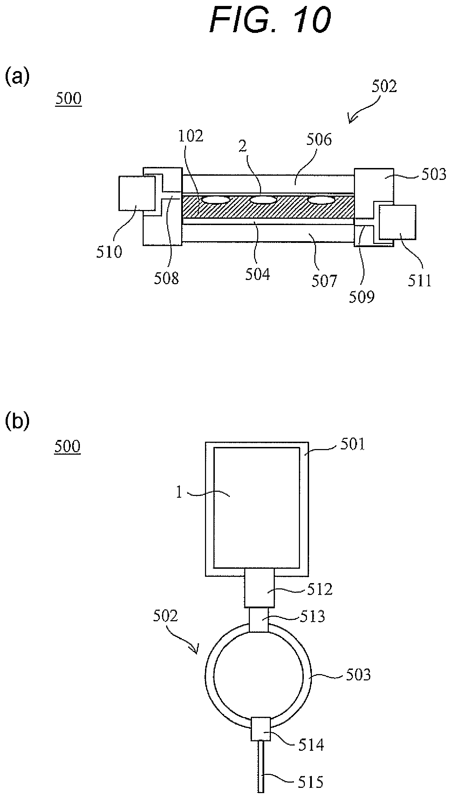

FIG. 10(a) is a schematic cross-sectional view illustrating a filter cartridge 502 of a cell detection device 500 according to the fifth embodiment. As illustrated in FIG. 10(a), the filter cartridge 502 includes a filter holder 503, a mesh 504, lids 506 and 507, flow paths 508 and 509, and plugs 510 and 511.

The filter 102 is fixed on the mesh 504 held by the annular filter holder 503. The mesh 504 serves to support the filter 102 when the strength of the filter 102 is low.

The filter holder 503 is provided with the flow path 508 and the flow path 509. The flow path 508 and the flow path 509 are sealed by the plugs 510 and 511, respectively, before the addition of the culture medium. The flow path 508 is provided on the bacteria capturing surface side of the filter 102 The flow path 509 is provided on the surface side of the filter 102 opposite to the bacteria capturing surface.

A funnel is attached to the bacteria capturing surface side of the filter 102. A filtration stage is attached to the mesh 504 side. A sample solution is supplied from the funnel. Due to this configuration, the sample can be filtered and the cells can be captured by the filter 102. After filtration of the sample, the disk-shaped lids 506 and 507 (members defining a space) are attached to both sides of the filter 102 so as to surround the filter 102. By providing the lids 506 and 507 in the filter holder 503, the filter 102 becomes the filter cartridge 502 (culture container) in a state of being sealed in a vessel having a small volume of about 2 times or less the filter volume.

FIG. 10(b) is a top view illustrating a configuration in which a culture medium is added to the filter cartridge 502.

As illustrated in FIG. 10(b), the plugs 510 and 511 that have sealed the two flow paths 508 and 509 are removed. The medium container 501 is connected to the flow path 508 via a check valve 513 and a liquid feeding pump 512. A capillary 515 is connected to the flow path 509 via a valve 514. The flow path 508 is connected to the bacteria capturing surface of the filter 102. The flow path 509 is connected to the opposite surface. The culture medium 1 is supplied to the bacteria capturing surface side of the filter 102 via the check valve 513 and the flow path 508 by the liquid feeding pump 512 to flow to the flow path 509 through the filter 102. That is, the flow direction of the culture medium 1 is substantially parallel to the filter surface. By flowing the culture medium substantially parallel to the filter surface, it is possible to spread the culture medium over the entire filter surface even with a small amount of culture medium as compared with the case of flowing the culture medium vertically.

### <Cell Detection Method>

The cell detection method using the cell detection device 500 of the present embodiment is substantially similar to the cell detection methods of the second to fourth embodiments described above. Thus, the cell detection method will be briefly described below. In the present method, the computing device actually drives each drive device to drive the liquid feeding pump 512 and the like. But, in order to simplify the description, the subject of the operation may be simply described as the computing device.

First, before performing the luminescence measurement, the computing device causes a sufficient amount of the culture medium 1 to flow by the liquid feeding pump 512 to clean the filter 102 and the flow paths 508 and 509 and to discharge the culture medium 1 from the capillary 515. Thereafter, for example, as in the fourth embodiment, the operation of cell detection can be executed using a plate having a plurality of wells (collection containers) into which the luminescent reagent has been dispensed in advance. Specifically, the computing device feeds a predetermined amount of the culture medium 1 from the medium container 501 into the filter holder 503 by driving the liquid feeding pump 512. The computing device collects the culture medium 1 from the capillary 515 into the first well, and measures luminescence by the luminescence measurement device. Thereafter, the computing device retracts the filter cartridge 502 from the first well. The computing device again feeds a predetermined amount of the culture medium 1 into the filter holder 503. The computing device, after culture for a predetermined time, collects the culture medium 1 from the capillary 515 into the second well to perform luminescence measurement by the luminescence measurement device. In this manner, the computing device repeats an operation of adding the culture medium 1 to the filter 102, culturing for a predetermined time and measuring luminescence.

### <Technical Effect>

As described above, the cell detection device 500 according to the fifth embodiment includes the filter cartridge 502 having a configuration in which the culture medium 1 is added from the direction substantially parallel to the surface of the filter 102. This makes it possible to efficiently collect a small amount of culture medium without retaining the culture medium in the peripheral edge portion of the filter even with a filter having a large area.

### [Sixth Embodiment]

It has been explained in the first to fifth embodiments that the cell detection device inspects the presence or absence of bacteria using the filter 102 obtained by filtering the sample solution. Since the bacteria detected by the method of each embodiment are non-destructive, they can also be used for subsequent analysis. Therefore, proposed is, in the sixth embodiment, a cell detection device capable of counting the number of colonies of detected bacteria.

### <Configuration of Cell Detection Device>

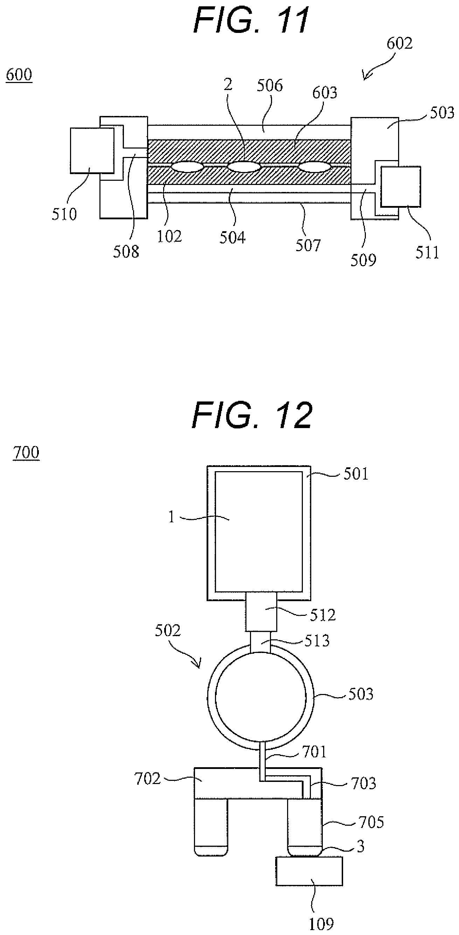

FIG. 11 is a schematic cross-sectional view illustrating a filter cartridge 602 of a cell detection device 600 according to the sixth embodiment. As illustrated in FIG. 11, the filter cartridge 602 (culture container) has substantially the same configuration as the filter cartridge (FIG. 10) of the fifth embodiment, but is different in that a fixing member 603 is adhered to the back surface of the lid 506. The fixing member 603 is in contact with the bacteria capturing surface of the filter 102. The bacteria 2 captured by the filter 102 are fixed by being sandwiched between the filter 102 and the fixing member 603. The internal volume of the filter cartridge 602 including the fixing member 603 may be within 5 times the volume (area × thickness) of the filter 102.

The material of the fixing member 603 is not particularly limited as long as it can fix the position of the bacteria 2 and does not affect the bacteria 2, for example, the same material as the filter 102, an agar culture medium (gel), or the like. When a transparent gel is used as the fixing member 603, after the bacteria 2 are detected using the cell detection device 600, the bacteria 2 are further cultured to form visible colonies. Then, the filter cartridge 602 is taken out, so that observation can be performed as it is.

### <Technical Effect>

As described above, the cell detection device 600 according to the sixth embodiment includes the filter cartridge 602 having a configuration in which the culture medium 1 is added from a direction substantially parallel to the surface of the filter 102. The fixing member 603 for fixing the position of the bacteria 2 is provided on the back surface of the lid 506 of the filter cartridge 602. As a result, viable bacteria counts can be counted by forming visible colonies by culturing after detecting bacterial positivity.

### [Seventh Embodiment]

In the fifth embodiment described above, the method has been described in which a filter cartridge incorporating a filter is used to collect a culture medium in different wells (collection containers) for each culture time to measure luminescence. In order to collect the culture medium in different wells, in the fifth embodiment, the filter cartridge 502 is moved to insert the capillary 515 into each well. On the other hand, in the seventh embodiment, proposed is a technique of collecting the culture medium in a plurality of wells without moving the filter cartridge 502.

### <Configuration of Cell Detection Device>

FIG. 12 is a schematic view illustrating a cell detection device 700 according to the seventh embodiment. As illustrated in FIG. 12, the cell detection device 700 includes a medium container 501 (medium container) for storing the culture medium 1, a liquid feeding pump 512 (medium adding device), a filter cartridge 502 (culture container), a flow path 701, a switching valve 702 (selection mechanism, medium collection device), and a plurality of wells 705 (a plurality of collection containers) for storing the luminescent reagent 3.

In the filter cartridge 502, a flow path on a side where the culture medium 1 is discharged is connected to the flow path 701. The flow path 701 is connected to the switching valve 702.

The switching valve 702 has a flow path 703 switched so as to communicate with each of the plurality of wells 705. Switching of the flow path 703 is controlled by a valve controller (not illustrated in FIG. 12) connected to the computing device.

### <Cell Detection Method>

The cell detection method using the cell detection device 700 of the present embodiment is substantially the same as the cell detection methods of the second to fourth embodiments described above, and thus will be briefly described below.

First, before performing the luminescence measurement, the computing device causes a sufficient amount of the culture medium 1 to flow by the liquid feeding pump 512 to clean and discharge the filter and the flow path 701. Next, the computing device connects the flow path 701 to the switching valve 702. Next, as in the fifth embodiment, the computing device feeds a predetermined amount of the culture medium 1 to the filter cartridge 502 for each culture time. After culture for a predetermined time, the computing device collects the culture medium 1 after culture in a different well 705 every time by switching the switching valve 702 to mix the culture medium 1 with a luminescent reagent. The computing device causes a luminescence reaction to perform luminescence measurement by the luminescence measurement device.

### <Technical Effect>

As described above, the cell detection device 700 according to the seventh embodiment includes the switching valve 702 that switches the flow path 703 such that the flow path 701 connected to the filter cartridge 502 communicates with one well 705 as a configuration for collecting the culture medium 1 into the wells 705 different for each culture time. As a result, it is possible to measure the luminescence for each culture time while keeping the inside of the medium container 501, the filter cartridge 502, and the well 705 hermetically sealed, so that it is possible to prevent foreign substances from being mixed from the outside during culture. In addition, since movement for retracting the filter cartridge 502 from each well 705 or inserting the flow path 701 into each well 705 becomes unnecessary, a space for moving the filter cartridge 502 is not necessary, and downsizing of the device can be realized.

### [Eighth Embodiment]

It has been explained, in the first to seventh embodiments described above, that the cell detection device has a configuration in which only one container (medium container) in which the culture medium is stored is provided and in which a predetermined amount of the culture medium is added to the filter. Proposed is, in an eighth embodiment, a cell detection device having a plurality of containers in which a culture medium is stored.

### <Configuration of Cell Detection Device>

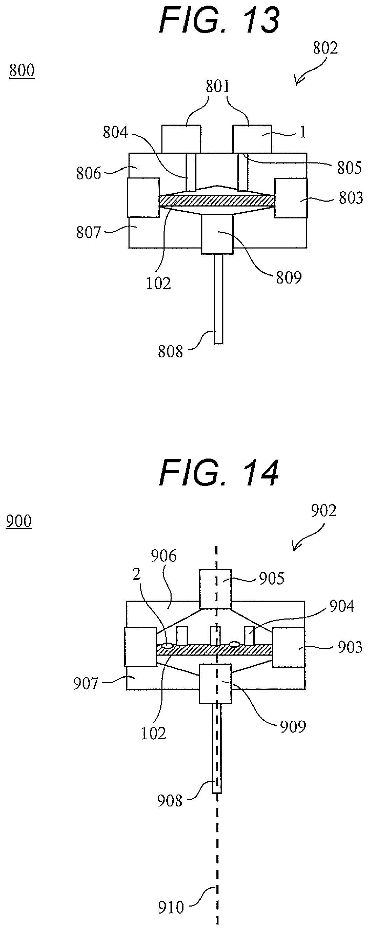

FIG. 13 is a schematic cross-sectional view illustrating a medium container 801 and a filter cartridge 802 of a cell detection device 800 according to an eighth embodiment. As illustrated in FIG. 13, the filter cartridge 802 (culture container) includes an annular filter holder 803 that holds the filter 102, adapters 806 and 807, a valve 809, and a capillary 808.

The filter holder 803 is sandwiched between the adapters 806 and 807 (members defining a space). Recesses are formed on surfaces of the adapters 806 and 807 facing the filter 102. As a result, a space in which the filter 102 is accommodated is formed. The adapter 806 is provided with two flow paths 804 that communicate the space with the outside. A medium container 801 (a plurality of medium containers) for storing the culture medium 1 is connected to each of the two flow paths 804. A valve 805 is provided at a boundary portion between the flow path 804 and the medium container 801. A space in which the filter 102 is accommodated is separated from the culture medium 1.

In the medium container 801, a previously calculated amount of the culture medium 1 necessary for one culture is sterilized and stored.

Each of the medium containers 801 can also contain a different type of culture medium. In this case, since it is possible to detect viable bacteria depending on the type of the culture medium, it is possible to classify the bacteria according to the culture medium in which the bacteria contained in the sample have been grown or have not been grown.

For example, a device (medium adding device) capable of compressing the medium container 801 (capable of applying a pressure to the medium container 801) is connected to the medium container 801. The culture medium 1 in the medium container 801 passes through the valve 805 and is added onto the filter 102 by applying a predetermined value or more of pressure to the medium container 801.

An adapter 227 is provided with the capillary 808 connected to a space in which the filter 102 is accommodated. The valve 809 is provided between the space in the adapters 806 and 807 and the capillary 808. The driving of the valve 809 is controlled by a drive device (medium adding device) (not illustrated).

### <Cell Detection Method>

The cell detection method using the cell detection device 800 of the present embodiment is substantially the same as the cell detection methods of the second to fourth embodiments described above, and thus will be briefly described below.

First, before luminescence measurement is performed, a sufficient amount of the culture medium 1 is caused to flow from the flow path 804 to clean and discharge the filter 102 and the capillary 808. Next, a medium container 801 is connected to each flow path 804. Next, as in the fifth embodiment, one medium container 801 is compressed for each culture time, and the entire amount of the culture medium 1 in the medium container 801 is fed to the filter cartridge 802 and cultured for a predetermined time. That is, a part of the total amount of the culture medium 1 in all the medium containers 801 is fed to the filter cartridge 802. Thereafter, the culture medium 1 after culture is collected in different wells each time. Then, the culture medium 1 is mixed with a luminescent reagent to cause a luminescence reaction to perform luminescence measurement.

### <Technical Effect>

As described above, the cell detection device 800 according to the eighth embodiment includes the plurality of medium containers 801 storing the culture medium 1 in an amount necessary for one culture. The cell detection device 800 is connected to the plurality of medium containers 801 and the filter cartridge 802. This makes it possible to easily add a small amount of culture medium to the filter 102 a plurality of times without requiring a precise mechanical mechanism.

### [Ninth Embodiment]

As described above, since the bacteria detected by the method of each embodiment are non-destructive, they can also be used for subsequent analysis. It has been explained, in the sixth embodiment, that the cell detection device is capable of counting the number of colonies of detected bacteria. Therefore, proposed is, in the ninth embodiment, another cell detection device capable of counting the number of colonies of detected bacteria.

### <Configuration of Cell Detection Device>

FIG. 14 is a schematic cross-sectional view illustrating a filter cartridge 902 of a cell detection device 900 according to the ninth embodiment. As illustrated in FIG. 14, the filter cartridge 902 (culture container) includes an annular filter holder 903 that holds the filter 102, a partition wall 904, adapters 906 and 907, valves 905 and 909, and a capillary 908.

The filter holder 903 is sandwiched between the adapters 906 and 907. Recesses are formed on surfaces of the adapters 906 and 907 facing the filter 102. As a result, a space is formed in which the filter 102 is accommodated. The valve 905 is provided in the recess of the adapter 906. A medium container (not illustrated in FIG. 14) is connected to the valve 905. As a result, the space in which the filter 102 is accommodated can communicate with the medium container. The valve 909 is provided in the recess of the adapter 907. The capillary 908 is connected to the valve 909.

The partition wall 904 is disposed on the cell-capturing surface of the filter 102. The partition wall 904 divides the cell-capturing surface of the filter 102 into a plurality of grids. The bacteria 2 in each grid cannot move to another grid.

The shape of the upper surface of the partition wall 904 is not particularly limited, and may be, for example, a lattice shape, a radial shape, a concentric shape, or any combination thereof. However, from the viewpoint of accurately counting the bacteria 2, the shape of the partition wall 904 can be selected so that the areas of the grids are substantially equal. The partition wall 904 can be made of resin, for example.

### <Cell Detection Method>

The cell detection method using the cell detection device 900 of the present embodiment is substantially the same as the cell detection methods of the second to fourth embodiments described above, and thus will be briefly described below.

First, in a state where the filter 102 is installed in the filter holder 903 and the partition wall 904 is disposed on the filter 102, the sample solution is added from above the filter 102 to perform filtration. At this time, the bacteria 2 contained in the sample are captured by any one of the grids.

Thereafter, the filter holder 903 is sandwiched between the adapters 906 and 907 to assemble the filter cartridge 902.

Then, upon addition of the culture media via the valve 905, the culture media can spread over the entire surface of the filter 102 due to the capillary action. Alternatively, the filter cartridge 902 can be rotated about a central axis 910 of the filter cartridge 902 to generate a centrifugal force from the center of the filter 102 toward the edge direction (radially outward), thereby spreading the culture medium over the entire surface of the filter 102.

Next, the added culture medium is collected from the valve 909 and the capillary 908 to perform luminescence measurement. As described so far, operations are repeated for addition of a culture medium, culture for a predetermined time, collection of the culture medium, and measurement of luminescence. When an increase in the luminescence amount is observed, it is determined that bacteria are positive.

Thereafter, by removing the adapter 907 from the filter cartridge 902, placing the filter 102 on an appropriate agar culture medium or liquid culture medium, and further culturing, it is possible to confirm the cell proliferation in each grid by visual or photometric means. When the number of cells contained in the original sample is smaller than the total number of grids, the number of grids in which cell proliferation is observed can be considered as the number of viable bacteria in the original sample.

### <Technical Effect>

As described above, in the cell detection device 900 according to the ninth embodiment is provided with the partition wall 904 that forms a grid on the cell-capturing surface on the filter 102. Thus, the viable bacteria count can be counted by further culturing after the detection of the bacteria.

### [Tenth Embodiment]

It has been explained, in the first to ninth embodiments, that the culture medium added to the filter is collected in the collection container disposed below the filter by centrifugation. A tenth embodiment proposes an example in which the collection container is disposed on substantially the same plane as the filter.

### <Configuration of Cell Detection Device>

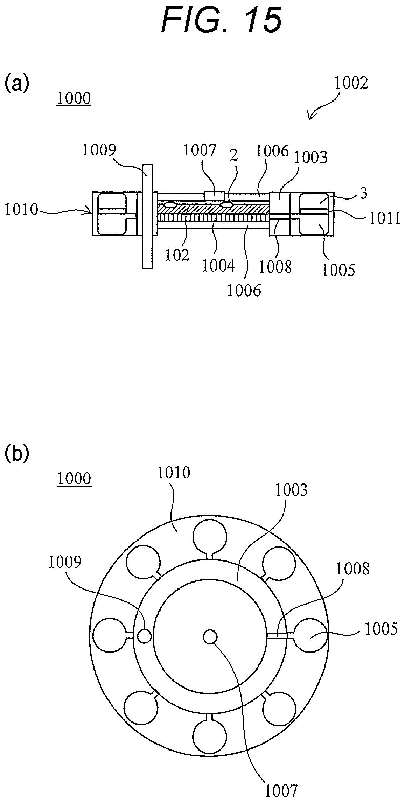

FIG. 15(a) is a schematic cross-sectional view illustrating a filter cartridge 1002 and a collection container cartridge 1010 of a cell detection device 1000 according to the tenth embodiment. As illustrated in FIG. 15, the collection container cartridge 1010 is formed in an annular shape and is provided around the annular filter cartridge 1002.

The filter cartridge 1002 includes an annular filter holder 1003 that holds the filter 102, upper and lower lids 1006 that seal the inside of the filter holder 1003, and a rotation shaft 1009 provided perpendicular to the filter 102 at a radial end of the filter holder 1003.

The filter holder 1003 has a mesh 1004. The filter 102 in which the bacteria 2 are captured is placed on the mesh 1004. A septum 1007 is provided in a central portion of the upper lid 1006. By causing the septum 1007 to penetrate a capillary connected to a container for storing the culture medium or a needle of a syringe for storing the culture medium, the culture medium can be supplied to a space in which the filter 102 is stored. The filter holder 1003 is provided with a flow path 1008 substantially parallel to the filter 102.

In the collection container cartridge 1010, a plurality of collection containers 1005 are formed along the circumferential direction of the collection container cartridge 1010. One of the collection containers 1005 communicates with the flow path 1008 (selection mechanism) of the filter holder 1003. The inner space of the collection container 1005 is vertically divided by a breakable seal 1011. The luminescent reagent 3 is accommodated on a seal 1011. A space below the seal 1011 communicates with the flow path 1008 of the filter holder 1003. For example, the collection container 1005 can be configured such that the upper surface on the side where the luminescent reagent 3 is stored can also be broken. As a result, for example, the upper surface of the collection container 1005 and the seal 1011 are broken by a needle penetrating therethrough. The luminescent reagent 3 can be added to the space below the seal 1011.

When the flow path 1008 and the collection container 1005 are communicated with each other, the collection container cartridge 1010 is rotated with respect to the filter cartridge 1002 to be aligned.

Although not illustrated, the cell detection device 1000 includes a rotation mechanism (medium collection device) that rotates the filter cartridge 1002 about the rotation shaft 1009 in a state where the filter cartridge 1002 and the collection container cartridge 1010 are assembled (in a state where the flow path 1008 and the collection container 1005 communicate with each other). The rotation about the rotation shaft 1009 applies a centrifugal force to the culture medium present in the space in which the filter 102 is accommodated. The culture medium moves in a direction away from the rotation shaft 1009.

FIG. 15(b) is a schematic top view illustrating the filter cartridge 1002 and the collection container cartridge 1010. As illustrated in FIG. 15(b), the flow path 1008 is provided on the opposite side of the rotation shaft 1009 by 180°. With such a positional relationship, the culture medium in the filter cartridge 1002 can be collected in the collection container 1005 with a high collection rate and a small residual liquid.

### <Cell Detection Method>

The cell detection method using the cell detection device 1000 of the present embodiment is substantially the same as the cell detection methods of the second to fourth embodiments described above, and thus will be briefly described below.

First, the sample solution is filtered by the filter 102 of the filter cartridge 1002 to capture the bacteria 2. Next, the lid 1006 and the rotation shaft 1009 are attached to the filter cartridge 1002 The collection container cartridge 1010 is attached around the filter cartridge 1002. Thus, the flow path 1008 communicates with the first collection container 1005.

Next, the culture medium is added from the septum 1007 into the filter cartridge 1002 using, for example, a syringe containing the culture medium. The filter cartridge 1002 is rotated about the rotation shaft 1009 by the rotation mechanism. The culture medium is collected in the space below the seal 1011 of the first collection container 1005.

Next, the collection container 1005 and the seal are broken by, for example, a syringe needle for breaking the collection container and the seal 1011 The culture medium collected in the first collection container 1005 is reacted with the luminescent reagent. A luminescence measurement device is disposed at a position where luminescence from the bottom surface of the collection container 1005 can be detected, to perform luminescence measurement.

Next, the collection container cartridge 1010 is rotated with respect to the filter cartridge 1002 to allow the flow path 1008 and the second collection container 1005 to communicate with each other.

Next, the culture medium is added from the septum 1007 into the filter cartridge 1002 using a syringe. After performing culture for a predetermined time, the filter cartridge 1002 is rotated about the rotation shaft 1009 by the rotation mechanism. Then, the culture medium is collected in the space below the seal 1011 of the second collection container 1005.

Hereinafter, luminescence measurement is performed in the same manner as in the first collection container 1005. As described so far, operations are repeated for addition of a culture medium, culture for a predetermined time, collection of the culture medium, and measurement of luminescence. When an increase in the luminescence amount is observed, it is determined that bacteria are positive.

### <Technical Effect>

As described above, in the cell detection device 1000 according to the tenth embodiment, the filter cartridge 1002 rotates about the rotation shaft 1009 provided in the peripheral portion of the filter cartridge 1002 in a state where the space in which the filter 102 is accommodated communicates with one collection container 1005. As a result, since the culture medium can be collected from the filter 102 to the collection container 1005 by the centrifugal force, a high collection rate can be achieved.

Further, when the culture medium is collected in the collection container 1005, it is not necessary to move the filter cartridge 1002 and the collection container cartridge 307 to a separately prepared centrifuge. This eliminates the need for a space for moving the filter cartridge 1002 and the collection container cartridge 1010, thereby achieving downsizing of the apparatus.

### [Modifications]

The present disclosure is not limited to the examples described above, but includes various modifications. For example, the above embodiments have been described in detail for easy understanding of the present disclosure, and thus the invention does not necessarily have all the configurations described. In addition, some of certain embodiment can be replaced with the configuration of the other embodiment. Further, it is possible to add the configuration of one embodiment to the configuration of another embodiment. It is also possible to add, delete, or replace a part of the configuration of another embodiment with respect to a part of the configuration of each embodiment.

### [Appendix]

### <Subject Matter 1>

A cell detection device including:
a medium adding device configured to add a part of a prepared culture medium to a culture container holding cells to be detected;
a medium collection device configured to collect the culture medium from the culture container; and
a detection device configured to detect light from the luminescent reagent mixed with the collected culture medium.

### <Subject Matter 2>

The cell detection device according to Subject Matter 1, wherein the culture container includes a filter having a pore diameter smaller than a size of the cell, and the cell is held by the filter.

### <Subject Matter 3>

The cell detection device according to Subject Matter 2, wherein a volume of the part of the culture medium added to the culture container by the medium adding device is 1/2 or more and 5 times or less of a value obtained by multiplying an area and a thickness of the filter.

### <Subject Matter 4>

The cell detection device according to Subject Matter 1, wherein
the luminescent reagent contains luciferase, and
the light is luminescence due to a reaction between adenosine triphosphate or luciferin generated in the culture medium from the cell and the luciferase.

### <Subject Matter 5>

The cell detection device according to Subject Matter 1, wherein the medium collection device further includes a selection mechanism configured to connect an arbitrary collection container among a plurality of collection containers from which the part of the culture medium is collected and the culture container.

### <Subject Matter 6>

The cell detection device according to Subject Matter 2, wherein the medium adding device adds the culture medium to the culture container substantially parallel to the filter surface.

### <Subject Matter 7>

The cell detection device according to Subject Matter 2, wherein the culture container includes a member covering a surface of the filter that holds the cell.

### <Subject Matter 8>

The cell detection device according to Subject Matter 1, wherein the medium adding device adds an entire amount of the culture medium in the medium container to the culture container from an arbitrary medium container among a plurality of medium containers each storing the part of the culture medium.

### <Subject Matter 9>

The cell detection device according to Subject Matter 1, wherein at least a part of a collection container that stores the culture medium collected from the culture container is configured to transmit the light from the luminescent reagent.

### <Subject Matter 10>

The cell detection device according to Subject Matter 1, wherein a medium container storing the prepared culture medium, the culture container and the collection container are disposed such that the culture medium moves in order of the medium container, the culture container and the collection container storing the culture medium collected from the culture container.

### <Subject Matter 11>

The cell detection device according to Subject Matter 1, further including a luminescent reagent adding device configured to add the luminescent reagent to the culture medium collected by the medium collection device.

### <Subject Matter 12>

The cell detection device according to Subject Matter 1, wherein the culture container and a collection container storing the collected culture medium are disposed at positions separated from each other in a same sealed space.

### <Subject Matter 13>

The cell detection device according to Subject Matter 2, wherein the culture container includes a filter holder configured to hold the filter, and a member configured to define a space for accommodating the filter.

### <Subject Matter 14>

The cell detection device according to Subject Matter 5, wherein the selection mechanism is a valve that switches a flow path for communicating the culture container with one of the plurality of collection containers.

### <Subject Matter 15>

The cell detection device according to Subject Matter 2, further including: a partition wall, disposed on a surface of the filter that holds the cell, for dividing the surface into a plurality of grids.

### <Subject Matter 16>

The cell detection device according to Subject Matter 5, wherein
the plurality of collection containers are disposed around the culture container,
the selection mechanism is a flow path configured to allow the culture container and one of the plurality of collection containers to communicate with each other, and
the medium collection device is configured to rotate the culture container in a state where the culture container communicates with one of the plurality of collection container.

### <Subject Matter 17>

A cell detection method including:
adding a part of a prepared culture medium to a culture container holding cells to be detected;
collecting the culture medium from the culture container; and
detecting light from a luminescent reagent mixed with the collected culture medium.

### <Subject Matter 18>

The cell detection method according to Subject Matter 17, further including:
repeating adding the culture medium, collecting the culture medium, and detecting the light a plurality of times; and
comparing the plurality of times of detection results.

### <Subject Matter 19>

The cell detection method according to Subject Matter 17, wherein
the culture container includes a filter having a pore diameter smaller than a size of the cell, and
the cell detection method further includes:
   capturing the cell in the filter.

### <Subject Matter 20>

The cell detection method according to Subject Matter 19, wherein a volume of the part of the culture medium added to the culture container is 1/2 or more and 5 times or less of a value obtained by multiplying an area and a thickness of the filter.

### <Subject Matter 21>

The cell detection method according to Subject Matter 18, wherein the culture medium is collected in a different collection container every time in the plurality of times of repeating.

### <Subject Matter 22>

The cell detection method according to Subject Matter 19, wherein the culture medium is moved to the culture container substantially parallel to the filter surface.

### <Subject Matter 23>

The cell detection method according to Subject Matter 19, further including:
providing a member configured to cover a surface of the filter that holds the cell in the culture container.

### <Subject Matter 24>

The cell detection method according to Subject Matter 17, wherein
the luminescent reagent contains luciferase, and
the light is luminescence due to a reaction between adenosine triphosphate or luciferin generated in the culture medium from the cell and the luciferase.

### <Subject Matter 25>

The cell detection method according to Subject Matter 18, wherein in the plurality of times of repeating, an entire amount of the culture medium is added to the culture container from a different medium container every time.

### <Subject Matter 26>

The cell detection method according to Subject Matter 18, further including:
determining that the cell is positive when it is determined that an amount of a substance derived from the cell has been increased by the comparing, and determining that the cell is negative when it is determined that the amount of the substance has not been increased.

### <Subject Matter 27>

The cell detection method according to Subject Matter 26, further including:
culturing the cell in the culture container after the cell is determined to be positive.

### Reference Signs List

- 1: culture medium
- 2: bacteria
- 3: luminescent reagent
- 100 to 1000: cell detection device
- 101: syringe
- 102: filter
- 103: filter holder
- 104: mesh
- 105: collection container
- 106: lid
- 107: septum
- 108: needle of syringe
- 109: luminescence measurement device

## Claims

1. A cell detection device comprising:
a medium adding device configured to add a part of a prepared culture medium to a culture container holding cells to be detected;
a medium collection device configured to collect the culture medium from the culture container; and
a detection device configured to detect light from luminescent reagent mixed with the collected culture medium.

2. The cell detection device according to claim 1, wherein the culture container includes a filter having a pore diameter smaller than a size of the cell, and the cell is held by the filter.

3. The cell detection device according to claim 2, wherein a volume of the part of the culture medium added to the culture container by the medium adding device is 1/2 or more and 5 times or less of a value obtained by multiplying an area and a thickness of the filter.

4. The cell detection device according to claim 1, wherein
the luminescent reagent contains luciferase, and
the light is luminescence due to a reaction between adenosine triphosphate or luciferin generated in the culture medium from the cell and the luciferase.

5. The cell detection device according to claim 1, wherein the medium collection device further includes a selection mechanism configured to connect the culture container and an arbitrary collection container among a plurality of collection containers from which the part of the culture medium is collected.

6. The cell detection device according to claim 2, wherein the medium adding device adds the culture medium to the culture container substantially parallel to the filter surface.

7. The cell detection device according to claim 2, wherein the culture container includes a member covering a surface of the filter that holds the cell.

8. The cell detection device according to claim 1, wherein the medium adding device adds an entire amount of the culture medium in the medium container to the culture container from an arbitrary medium container among a plurality of medium containers each storing the part of the culture medium.

9. The cell detection device according to claim 1, wherein at least a part of a collection container that stores the culture medium collected from the culture container is configured to transmit the light from the luminescent reagent.

10. The cell detection device according to claim 1, wherein a medium container storing the prepared culture medium, the culture container and the collection container are disposed such that the culture medium moves in order of the medium container, the culture container and the collection container storing the culture medium collected from the culture container.

11. A cell detection method comprising:
adding a part of a prepared culture medium to a culture container holding cells to be detected;
collecting the culture medium from the culture container; and
detecting light from a luminescent reagent mixed with the collected culture medium.

12. The cell detection method according to claim 11, further comparing:
repeating adding the culture medium, collecting the culture medium, and detecting the light a plurality of times; and
comparing the plurality of times of detection results.

13. The cell detection method according to claim 12, wherein the culture medium is collected in a different collection container every time in the plurality of times of repeating.
